# EUROPEAN PATENT SPECIFICATION

(11) **EP 1 505 076 B1**
(45) Date of publication and mention of the grant of the patent: **08.04.2009**
(21) Application number: 03720119.1
(22) Date of filing: 28.04.2003
(51) Int. Cl.: C07K 16/30, A61K 51/10

(54) **SPECIFIC ANTIBODY FRAGMENTS FOR THE HUMAN CARCINOEMBRYONIC ANTIGEN (CEA)**
SPEZIFISCHE ANTIKÖRPERFRAGMENTE FÜR DAS HUMANE CARCINOEMBRYONIC ANTIGEN (CEA)
FRAGMENTS D'ANTICORPS SPECIFIQUES POUR L'ANTIGENE CARCINOEMBRYONAIRE (ACE) HUMAIN

(30) Priority: 29.04.2002 CU 8602
(43) Date of publication of application: 09.02.2005
(73) Proprietor: CENTRO DE INGENIERIA GENETICA Y BIOTECNOLOGIA (CIGB), Ciudad de la Habana 10600 (CU)
(72) Inventor: Gavilondo Cowley, Jorge Victor, 10400 Ciudad de La Habana (CU); Ayala Ávila, Marta, C/ 186, No. 3117 Apto 3D e/, 10600 Ciudad de La Habana (CU); Freyre Almeida Freya, de los Milagros, Playa, 11600 C. Habana (CU); Acevedo Castro, Boris E., Calle Segunda, 12000 Ciudad de La Habana (CU); Bell Garcia, Hanssel, C/62 00906, 2do piso, apt 16, 10600 Ciudad de la Habana (CU); Roque Navarro, Lourdes Tatiana, 11300 Ciudad de la Habana (CU); Gonzalez Lopez, Luis Javier, Calle 186, 003115, 10600 Ciudad de la Habana (CU); Cremata Alvarez, José Alberto, Ave 31 No. 18207, 11600 Ciudad de la Habana (CU); Montesino Segui, Raquel, Calle 184 No. 3112, 11600. Ciudad de la Habana (CU)
(74) Representative: van Loon, C.J.J.
(86) International application number: PCT/CU2003/000005
(87) International publication number: WO 2003/093315

(56) References cited:
- SE-HO KIM ET AL.: "Characterization of Monoclonal Antibodies Against Carcinoembryonic Antigen (CEA) and Expression in E. coli" HYBRIDOMA, vol. 20, num. 4, Agosto 2001 (2001-08), XP002251700
- HOLLIGER P. ET AL.: ""Diabodies": Small bivalent and bispecific antibody fragments" PROC. NATL. ACAD. SCI., vol. 90, Julio 1993 (1993-07), XP002251701

## Description

### TECHNICAL FIELD

The present invention is related to the field of Immunology, and in particular it refers to antibody fragments of the single chain Fv type, in its mono- and divalent (diabody) forms, obtained by recombinant DNA techniques starting from a mouse monoclonal antibody of proven clinical efficacy, that is specific for the human carcinoembryonic antigen.

### BACKGROUND OF THE INVENTION

The carcinoembryonic antigen (CEA) is a 180 kDa glycoprotein, secreted preferably by the cells of gastrointestinal human tumors and other carcinomas even though it can also be detected in some non-malignant tissues as the colonic mucosa. Its physiological role has not been totally elucidated, and up to the moment it is believed it is associated in some way to the processes of cell adhesion (Gold P, Freedman SO. Journal of Experimental Medicine 122: 467: 1965; Zimmermann W et al. PNAS USA 84: 2960-2964; 1987; Paxton RJ et al. PNAS USA 84: 920-924, 1987; Beauchemin N et al. Molec. Cellular Biol. 7: 3221-3230, 1987; Gold P, Goldenberg NA. MJM 3:46-66,1997).

The CEA is a member of the immunoglobulin superfamily, due to its structure characterized by repetitive domains (Oikawa S et al. BBRC 144: 634-642, 1987; Thompson J, Zimmermann W. Tumor Biology 9: 63-83, 1988; Hammarstrom S. Seminars in Cancer Biology 67-81, 1999). The CEA has a high homology with other molecules of this superfamily, such as the NCA, the meconium antigen, the Biliar Glycoprotein type A, and the Glycoprotein b specific of pregnancy (von Kleist S, Burtin P. Immunodiagnosis of Cancer. Marcel Dekker. 322-341, 1979; Buchegger, F. et al. Int. J. Cancer 33; 643-649, 19984; Matsuoka Y et al. Cancer Res. 42:2012-2018, 1982; Svenberg T. Int. J. Cancer 17:588-596, 1976).

The elevation of circulating CEA levels is considered since many years ago as one of the best indicators of a possible relapse and/or metastases, in patients submitted to surgery for primary colorectal tumors that express this antigen (Gold P, Goldenberg NA. MJM 3:46-66, 1997). The measurement of circulating CEA has extended also as a method for the follow-up of other human carcinomas (breast, lung), in the cases in which significant pre-surgery levels of this tumor marker have been demonstrated (Gold P, Goldenberg NA. MJM 3:46-66, 1997). Since the discovery of the technology for generation of monoclonal antibodies (Mab; Kohler G, Milstein C. Nature 256:52-53, 1975), the immunoassays for the measurement of circulating CEA have improved in specificity and their use has widely extended.

The CEA has been studied also since many years ago as a possible "cell target" in order to specifically direct radioactive isotopes for *in vivo* diagnostics (Goldenberg DM Int. J. of Biol. Markers 7; 183-188, 1992) and *in situ* radiotherapy (Ledermann et al., Int. J. Cancer 47; 659-664, 1991). Its use has also been foreseen to target toxins, drugs, and other bioactive products towards the tumor cells (Bagshawe KD. Drug Dev. Res. 34:220-230, 1995).

The anti-CEA antibodies have been the main vehicles used for such purposes, starting with polyclonal preparations, to be followed later on by mouse Mab, their Fab fragments, antibody fragments obtained by genetic engineering from mouse Mab, and more recently, from libraries of murine and human antibodies displayed in filamentous phage (Hammarstrom S et al. Cancer Res. 49, 4852-4858, 1989; Hudson PJ Curr. Opinion Immunology 11:548-557, 1999; Griffiths AD et al. EMBO J. 12, 1993; 725-734; Griffiths AD et al. EMBO J. 13 3245-3260, 1994; WO93/11236; Chester K et al 1995, WO 95/15341; Allen DJ et al. 1996, US5872215).

The expression of antibodies and antibody fragments in prokaryotic cells like *E. coli,* and in other microorganisms is well established in the art (Pluckthun, A. Bio/Technology 9: 545-551, 1991; Gavilondo J, Larrick JW. Biotechniques 29: 128-132, 134-136, 2000). The expression of antibodies and antibody fragments, in superior eukaryotic cells in culture is also know for those skilled in the art (Reff ME. Curr. Opinion Biotech. 4: 573-576, 1993; Trill JJ et al. Curr. Opinion Biotech 6: 553-560, 1995).

Kim et al. (Hybridoma 20(4), 2001, pp. 265-271) characterize monoclonal antibodies against CEA produced in cell lines obtained by fusion of spleen cells from CEA-immunized Balb/C mice and SP2/O myeloma cells.

The mouse Mab denominated indistinctively as CB-CEA.1 or ior-CEA.1 (referred hereafter as CB/ior-CEA.1) is known from the state of the art. This Mab has a high specificity for human CEA, has no undesired cross-reactions with molecules such as NCA, nor recognizes normal tissues, exception made of the cells of the normal colon epithelium, where CEA can be commonly found polarized (Tormo B et al. APMIS 97: 1073-1080, 1989). This Mab has very high affinity for CEA (Pérez L et al. Applied Biochem. Biotechnol. 24: 79-82, 1996). This Mab labeled with ^{99m}Tc has been successfully employed in the diagnosis and follow up of human colorectal tumors. The clinical studies of radioimmunodetection showed that it has 91.3% sensitivity, 77.1% specificity, and 82.8% of positive predictive value (Oliva JP et al. Rev Esp Med Nucl. 13:4-10, 1994). This makes it superior in performance with respect to the only other anti-CEA monoclonal antibody employed clinically in the World at present for such purposes, the CEA-Scan (^{99m}Tc-Arcitumomab) from Immunomedics (Morris Plains, NJ, USA).

The development of a single chain Fv (scFv) antibody fragment, obtained through the polymerase chain reaction (PCR) from RNA extracted from the hybridoma that produces the Mab CB/ior-CEA.1, was reported in 1992 (Ayala M et al. Biotechniques 13: 790-799, 1992). In the experimental strategy followed, the amplification of the variable domains of CB/ior-CEA.1 was done with degenerate oligonucleotides for the framework regions of both variable domains. The scFv was produced in *E. coli* and demonstrated recognition of CEA in ELISA and in cytochemistry studies, but with a affinity for the immobilized antigen 200 times lower than the Fab obtained by the natural way (Pérez L et al. Applied Biochem. Biotechnol. 24: 79-82, 1996). This same fragment scFv was cloned, expressed, and produced in *Pichia pastoris* (Freyre FM et al. J Biotechnol. 76(2-3):157-163, 2000) without any improvements in affinity for human CEA, and the studies conducted in experimentation animals with the radiolabeled fragment indicated an anomalous biodistribution (Pimentel GJ et al. Nud Med Commun. 22:1089-94, 2001), that motivated not to continue its further development

### DESCRIPTION OF THE INVENTION

The present invention refers to single chain Fv (scFv) antibody fragments as defined in claim 1, in its mono- and divalent (diabody) forms. These may be obtained by DNA recombinant techniques starting from the anti-carcinoembryonic antigen (CEA) monoclonal antibody CB/ior-CEA.1 (Tormo B et al. APMIS 97: 1073-1080, 1989). This Mab has very high affinity for CEA (Pérez L et al. Applied Biochem. Biotechnol. 24: 79-82, 1996) and has been successfully employed in the diagnosis and follow-up of human colorectal tumors (Oliva JP et al. Rev Esp Med Nud. 13:4-10, 1994). The scFv monovalent and diabody fragments reported in the present invention can be produced through their expression in recombinant microorganisms, as bacteria and yeast. As the original Mab, the scFv monovalent and diabody fragments are specific for an epitope of human CEA that depends on the conservation of the carbohydrates and exhibit high affinities for this antigen. The scFv monovalent and diabody fragments have a recognition pattern *in vitro* of human normal and tumor cells and tissues similar to the original Mab and, as this, once radiolabeled, they have the capacity to identify tumor cells that express human CEA growing in athymic congenital mice. The scFv monovalent and diabody fragments have no Fc domains and have lower molecular size that the mouse Mab, this conferring them the potential to better penetrate the tissues *in vivo* and to be less immunogenic when applied to humans for diagnostic or therapeutic purposes.

The scFv monovalent and diabody fragments reported in this invention have important differences in aminoacids in the heavy chain (VH, and light chain (VL) variable domains, with respect to other scFv previously developed from the same Mab, and surpass it in affinity for CEA, in performance for the recognition of cells and tissues, and in efficacy for the localization of tumors that produce human CEA growing *in vivo* in mice.

The recombinant scFv monovalent and diabody fragments reported in this invention were developed using PCR, and cloning and expression techniques in recombinant microorganisms, starting from the RNA extracted from the CB/ior-CEA.1 hybridoma. Sets of oligonucleotides different from those used to obtain a previously reported scFv (Ayala et al. Biotechniques 13: 790-799, 1992), were employed for the amplification and isolation of the base sequences encoding the Mab VH and VL domains. In the invention it is shown that the new monovalent and diabody scFv have important differences in the aminoacid sequences of the VH and VL domains, with respect to a scFv previously obtained, and that these take the form of 16 aminoacids in the frameworks 1 (FR1) and 3 (FR3) and in the complementary determinant region 2 (CDR2) of the VH domain, different with respect to the scFv previously obtained, and 3 aminoacids between the FR1 and

FR3 of the VL domains, different with respect to the scFv previously obtained. This indicates that these domains have a different clonal origin with respect to those reported in Ayala et al. Biotechniques 13: 790-799, 1992. In the case of the diabody, this one also differs from the scFv previously obtained in the size and aminoacidic composition of the union segment (linker) that is employed in the fabrication of the scFv-type molecule.

The changes reflect surprisingly in the biochemical and biological properties of the new fragments, and provide them with a behavior very similar to the Mab CB/ior-CEA.1, and very much superior to that of the previously reported scFv. The new monovalent scFv fragment, that has a linker identical to the previously reported scFv (Ayala et al. Biotechniques 13: 790-799, 1992), but the aforementioned aminoacid changes in the variable domains, has an affinity constant for human CEA very much higher that the previously reported scFv.

Also, the diabody surpasses both monovalent scFv forms in its affinity constant for human CEA. The two new scFv monovalent and diabody fragments conserve the properties of specificity of the original Mab with respect to CEA recognition, identification of tumor cells and tissues, absence of cross reactivity with NCA, and capacity to accumulate selectively in a tumor that produces human CEA transplanted in mice, all with a very much superior performance than that of the previously obtained scFv.

The two new monovalent and diabody scFv have molecular sizes at least 5 and 2.5 times lower than the original Mab, respectively, a fact that confers these with the potential to better penetrate tissues and to be less immunogenic in the human being, all of which makes them more attractive and presumably superior that the original CB/ior-CEA.1 Mab to direct radioisotopes, drugs, toxins, and other bioactive elements to tumors that express human CEA.

In the present invention it is shown how it is possible to amplify by PCR the VH and VL domains of the Mab CB/ior-CEA.1 using synthetic oligonucleotides that hybridize in the base sequences that encode for the signal peptides and constant domains CH1 and Ck. It is also shown the possibility of assembly of the amplified VH and VL domains, in this order, using PCR, and obtaining different forms of scFv fragments manipulating the size of the linker that connects the domains. Using 14 aminoacids a monovalent scFv form is originated, and reducing this number to five, a diabody scFv type form is produced.

It is demonstrated in the invention that it is possible to express the monovalent and divalent scFv fragments in the bacteria E. coli and in the yeast *Pichia pastoris,* and that these fragments identify *in vitro* the human CEA , linked or not to tumor cells, in a specific manner. In the present invention it is also demonstrated that the radiolabeled monovalent and diabody scFv identify *in vivo* tumor cells that express human CEA and that grow as tumors in mice, exhibiting a behavior very similar to that of the Mab CB/ior CEA.1, and a performance very superior to the previously obtained scFv. In the present invention methods to purify and characterize the new scFv monovalent and diabody fragments, are also shown.

The antibody fragments described in this invention are useful to be applied in the diagnosis and therapy of cancer, with the advantages that these derive from a Mab of proved clinical efficiency, and that their lower size and absence of Fc domain allow both a better tissue penetration, and their use in repeated treatments due to the lesser capacity of induction of a human anti-mouse immunoglobulin response (HAMA; Schroff et al. Cancer Res 45: 879-885, 1985; DeJager et al. Proc. Am. Assoc. Cancer Res. 29:377, 1988). The HAMA responses are inconvenient for the treatment because of the neutralization of the biological effect of the administered antibody, the consequent dose lowering, and because these can cause allergic responses, "serum" sickness, and kidney affections.

### TERMINOLOGY

### Antibodies and their Specific Fragments

The terms describe an immunoglobulin of parts thereof with antigenic specificity, being these natural or produced partially or fully in a synthetic way. The terms also cover any polypeptide or protein that has a binding domain that would be the binding site of the antibody, or homologous to it. These can be produced naturally or in a synthetic way, either partially or fully. Examples of antibodies are the different classes and subclasses of immunoglobulins, and fragments of these that contain one or more antigen binding sites, such as Fab, scFv, Fv and the diabodies.

The antibodies and antibody fragments include any polypeptide that comprises an immunoglobulin binding domain, being this natural or produced synthetically, both fully or partially, and chimeric molecules that comprise an immunoglobulin binding domain, or its equivalent, fused to other polypeptide.

It has been shown that the fragments of a complete antibody can carry out the function of binding antigens. Examples or these binding fragments are: (i) the Fab fragment that includes the VL, VH, CL and CH1 domains of an immunoglobulin; (ii) the Fd fragment, that consists of the VH and CH1 domains; (iii) the Fv fragment, that consist in the VL and VH domains of a given antibody; (iv) the scFv fragment, where the VH and VL domains of a given antibody are united with a peptidic linker that allows the two domains to associate to form an antigen binding site (Bird et al, Science 242: 423-426, 1988; Huston et al, PNAS USA 85: 5879-5883, 1988); (v) "diabodies", multivalent or multispecific fragments constructed in a similar way to scFv but where the small size of the linker does not allow the VH and VL domains of the same scFv molecule to associate among them, and the antigen binding sites form through the association of two or more scFv (WO94/13804; Holliger P et al. PNAS USA 90 6444-6448, 1993); (vi) other fragments as the dAb (Ward SE et al., Nature 341: 544-546, 1989), isolated CDR regions, F(ab')₂ fragments and bispecific scFv dimers (PCT/US92/09965; Holliger P, Winter G. Current Opinion Biotechnol. 4: 446-449, 1993; de Haard, H et al. Adv. Drug Delivery Rev. 31:5-31, 1998).

The diabodies and scFv can be constructed without Fc regions, using only the variable domains, potentially reducing the effects of anti-isotype reactions when administered to humans. They are also particularly useful due to their production in *E. coli* and recombinant yeast. Their size inferior to that of a full immunoglobulin provides them with increased tissues penetration potential.

### Antigen binding site

This term described the part of an antibody that comprises the area that specifically interacts with all the antigen, or part of it. When the antigen is large, an antibody can only bind to a particular part of the antigen, denominated epitope. An antibody-binding site can be given by one or more antibody variable domains. Preferably, an antigen-binding site comprises the variable region (or domain) of the light chain (VL) and the variable region (or domain) of the heavy chain (VH) of an antibody.

### Specific

Refers to the situation in which an antibody or its fragment does not present a significant binding to other molecules different from its specific binding pair. This term is also applicable to the case where an antigen binding site is specific for a particular epitope that appears in a number of related or un-related antigens, in which case the binding site would be capable of binding to several antigen that bear the epitope.

### DETAILED DESCRIPTION OF THE INVENTION

Through the present invention, specific polypeptide molecules are obtained, formed by one or more antigen binding sites, coming from a mouse Mab that is specific for human CEA. The antigen binding site is assembled in the form of monovalent, divalent, and other forms of antibody fragments, depending on the way the polypeptide molecule is constructed.

The polypeptide molecule in the form of a monovalent scFv fragment specific for human CEA exhibits an affinity constant for this antigen of (5.0 ± 0.4) x 10⁹ L mol⁻¹, and comprises the VH and VL domains, linked in this order by a 14-aminoacid union segment (linker), with an aminoacid sequence as the one presented in SEQ ID No. 16.

The polypeptide molecule in the form of a divalent scFv fragment (diabody) specific for human CEA exhibits an affinity constant for this antigen of (2.8 ± 0.3) x 10¹⁰ L mol⁻¹, and comprises the pairing of two identical molecules formed each one by the VH and VL domains, linked in this order by a five-aminoacid union segment (linker), with an aminoacid sequence as the one presented in SEQ ID No. 17.

In another aspect of the invention, the monovalent and diabody scFv fragments do not bind, or bind in a non significant manner, with normal tissues, or cells from the following normal tissues: liver, kidney, lung, testicle, blood, spleen, and pancreas. In the case of the colon mucosa, the monovalent and diabody scFv fragments react exclusively with the products of luminal secretion and in apical zones or some glands. The absence of reactivity of the monovalent and diabody scFv fragments with normal lymphocytes and neutrophils is indicative that there is not an important level of cross reactivity with the NCA antigen (von Kleist S, Burtin P. lmmunodiagnosis of Cancer. Marcel Dekker. 322-341, 1979; Buchegger, F. et al. Int. J. Cancer 33; 643-649, 1984).

The monovalent and diabody scFv fragments can bind to soluble CEA, CEA absorbed to solid surfaces, or CEA associated to cells that produce it, and to tumor tissues, among which human colorectal, breast, lung, pancreas and stomach adenocarcinomas stand out. The monovalent and diabody scFv fragments and the Mab CB/ior-CEA.1. bind to soluble and solid surface bound CEA in a form that is dependent of the conservation of glycosylation of human CEA, suggesting that the carbohydrates of this antigen are involved in the recognition.

Polypeptide molecules derived from the monovalent and diabody scFv fragments reported in this invention, that retain the capacity of binding CEA, their reported affinity, specific epitope recognition, and similar and equivalent biological and biochemical performance to the fragments described in this invention are considered equivalent variant forms and are contained in the present invention. These polypeptide molecules can take the form of other recombinant antibody fragments, such as scFv where the VL domain precedes the VH; or Fab, Fab', F(ab')2, Fabc, Facb, trimeric and tetrameric scFv, etc. (Winter G, Milstein C. Nature 349: 293-299,1991; WO94/13804 de Haard, H et al. Adv. Drug Delivery Rev. 31:5-31, 1998), and other union segments (linkers) known in the state of the art are used. These can also be in the form of bi-specific antibody molecules, where a portion of such conserve specificity for CEA, and the other has a different specificity.

Equally contained in the present invention are the variant forms of the monovalent and diabody scFv fragments that comply with the characteristics described in the previous paragraphs, and that would have been derived from the so-called "humanization by immunogenicity reduction", in which B and T cell epitomes present in variable domains are modified in a way that the antigenic recognition is not altered, but the immunogenicity of the resulting molecule in humans is reduced, for example, as it is revealed in Carr FJ et al. 2000 EP 983303A1 and in Rodriguez Perez R et al. US 5712120-A. Equally considered variant forms contained in this invention are those produced by the so-called "CDR transplant" in which the CDR sequences of a first antibody are placed within the frame of sequences that are not from this antibody, for example, as it is revealed in EP-B-0239400, EP-A-184187, GB 2188638A or EP-A-239400, and retain the capacity of binding CEA with similar affinity, competitive capacity, particular epitope recognition, and biological and biochemical performance similar and equivalent to the monovalent and diabody scFv fragments described in this invention.

Apart from the antibody sequences, the polypeptide molecules contained in this invention can comprise other aminoacids that form a peptide or polypeptide, or that add to the molecule a functional characteristic different to that of binding the CEA antigen, as for example a tag for purification or identification, an enzyme or its fragments, a biological response modifier, a toxin or drug, and successively. In agreement with this invention the monovalent and diabody scFv fragments can be administered in isolated or purified form.

The present invention foresees the use of some of the polypeptide molecules described above as a diagnostic reagent for human cancer forms that express CEA, as for example, colon, lung, breast or other adenocarcinomas.

The polypeptide molecules specific for CEA describe above can be radiolabeled wand employed as agents to obtain images to demonstrate in a specific way the presence and location of tumors that express CEA in humans. The present invention provides a method to determine the presence of a cell or tumor that express CEA, being such method that of placing in contact the cells with a polypeptide molecules as the ones described, and determining the binding of these to the cells. The method can be developed *in vivo,* or in a sample of cells removed from the body, being this *in vitro* or *ex vivo.*

The present invention provides a method fro the binding of a polypeptide molecule as the ones described before, to human CEA. This binding can happen *in vitro, ex vivo* or *in vivo.* If the binding is in vivo, the method can comprise the administration of the polypeptide molecule to mammals, being these one or several individuals. As t is demonstrated experimentally here, the monovalent and diabody scFv fragments in this invention bind to human CEA expressed by transfected mouse tumor cells, which grow as tumors once they are transplanted to mice, providing an experimental model useful for the study, the investigation, and the development of molecules with specific binding and of their properties. The reactivity of the antibodies on cellular samples can be detected through any appropriate mean. Labeling with individual reporter molecules is one such possibility. Reporter molecules can generate signals capable of being detected directly or indirectly and preferably measured. The coupling of the reporter molecules can be direct or indirect, covalent or non covalent. The union through a peptide bond can result from the recombinant expression of a gene fusion that couples the antibody and the reporter molecule. The form of determining the coupling is not a characteristic of the present invention, and those skilled in the art are capable of choosing an adequate model in accordance to their preference and general knowledge.

When a radionuclide as ¹²⁵I, ¹¹¹In or ^{99m}Tc is used to label the monovalent and diabody scFv fragments and its equivalent forms, if these locate preferably in the tumor, and not in the normal tissues, the presence of he radioactive labeling in the tumor tissue can be detected and quantitated using a gamma camera. The quality of the obtained image of the tumor correlates directly with the ratio signal:background (Goldenberg DM. Int. J. of Biol. Markers 1992, 7; 183-188). The experimental use of ¹²⁵I is exemplified in the text.

The present invention also offers elements so that the monovalent and diabody scFv fragments and their equivalent variant forms as described before, can be used as a therapeutic reagent, fro example, when they are coupled, conjugated or bound to molecules with therapeutic power, or are generated as a recombinant fusion protein. The monovalent and diabody scFv fragments and their equivalent variant forms according to the present invention can be used to direct a toxin, radioactivity, T and NK cells, or other molecules to tumors that express CEA, or to develop an anti-idiotypic response in the organism that could conduct to a desired therapeutic effect. In agreement with this, other aspects of the invention provide elements for methods of treatment that involve the administration of monovalent and diabody scFv fragments or their equivalent variant forms, as medicaments or pharmaceutical compositions.

In agreement with the present invention, the compositions can be administered to individuals, preferably in a "therapeutically effective" amount, sufficient to demonstrate a benefit for the patient, in the way of the improvement of at least one symptom. Details related to the amount to administer, the frequency and intervals of administration will depend on the nature and severity of the disease that is treated, and these decisions are the responsibility of specialists and other medical doctors. The appropriate doses of an antibody are well known in the art (Ledermann J. A. et al. Int J. Cancer 47: 659-664, 1991; Bagshawe KD et al. Antibody, Immunoconjugates, and Radiopharmaceuticals 4: 915-922, 1991).

A composition can be administered alone or in combination with other treatments, either simultaneously or sequentially, depending of the disease to be treated.

The pharmaceutical compositions in agreement to the present invention, and to be employed according to the present invention, can comprise, apart from the active ingredient, an excipient, buffer, stabilizer or accepted pharmaceutical carrier, or other materials well known for those skilled in the art. These materials should not be toxic, should not interfere with the efficacy of the active ingredient and their precise nature could depend on the administration route, being this oral, or by injection, for example, intravenously.

The scFv monovalent and diabody fragments and their equivalent variant forms in agreement with the present invention can be fabricated through the expression of the encoding nuclei acid. The nucleic acid that encodes for any of these polypeptide molecules described before is part of the present invention, as it is a method for the expression of such nucleic acid. In a different embodiment, the nucleic aid can encode for the aminoacid sequences shown in SEQ ID No. 16 and 17.

For the recombinant expression of the monovalent and diabody scFv and their equivalent variant forms, appropriate vectors can be selected or constructed, with the adequate regulatory sequences, including promoter, terminator, enhancer, polyadenilation, marker genes, and other pertinent sequences. The vectors can be plasmids. Many known protocols and techniques for the manipulation of nucleic acids, for example, preparation of nuclei acid constructions, polymerase chain reaction, mutagenesis, sequencing, introduction of DNA in cells and gene expression, protein analysis, and others are described in detail in several references, as Molecular Cloning: a Laboratory Manual: 2nd edition, Sambrook et al., Cold Spring Harbor Laboratory Press, 1989 or Short Protocols in Molecular Biology, Second Edition, Ausubel et al. eds., John Wiley & Sons, 1992 or Erlich HA PCR Technology, Stockton Press, 1989.

Another aspect of the present invention provides a host cell containing a foreign nuclei acid and the methods to introduce such nucleic acid in a host cells. The introduction can employ any of the techniques that exist for such purpose. For bacterial and yeast cells, this technique can be the electroporation. The introduction can be followed by provoking or allowing the expression of the nucleic acid, for example, growing the host cells under conditions favorable for the expression of the gene. In one embodiment, the nucleic acid of the invention integrates in the genome of the host cells.

After their production, the monovalent and diabody scFv fragments and their equivalent variant forms, can be used in any of the forms revealed here, such as in the formulation of a composition as a pharmaceutical or a diagnostic product, such as in a set of reagents that comprises apart from the specific binding member, one or more reagents to determine the binding of the member to cells or to CEA not linked to cells, as discussed before.

Other further aspects of this invention and its realizations will be apparent to those experts in the art. For the complete understanding of this invention, and not to limit its extension and reach, examples are provided. Reference is made to the following figures:

### SHORT DESCRIPTION OF THE FIGURES

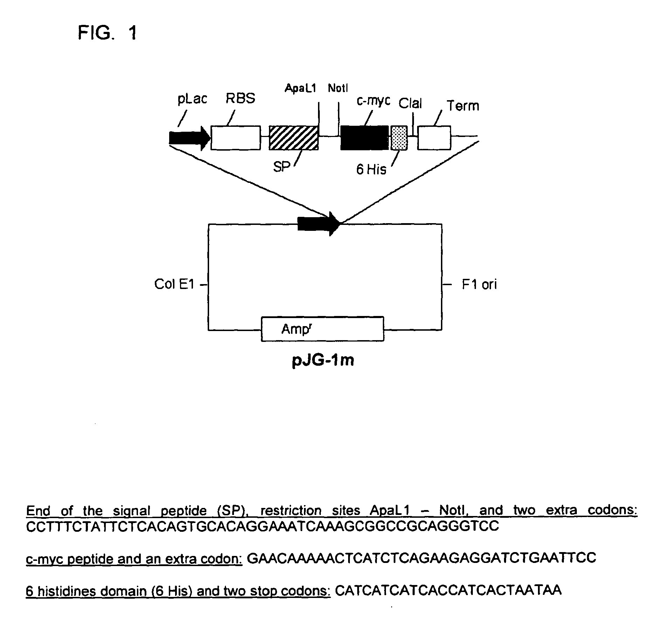

In FIGURE 1, a scheme of the pJG-1m vector used for the expression of the monovalent scFv and the diabody in E. coli is represented. In correspondence with the vector zone marked with the wide horizontal bar, the base sequences of the fragments cloning site, the c-myc peptide, the 6-hitidine domain, and some inter and pos-domain regions are presented (SEQ ID No. 13).

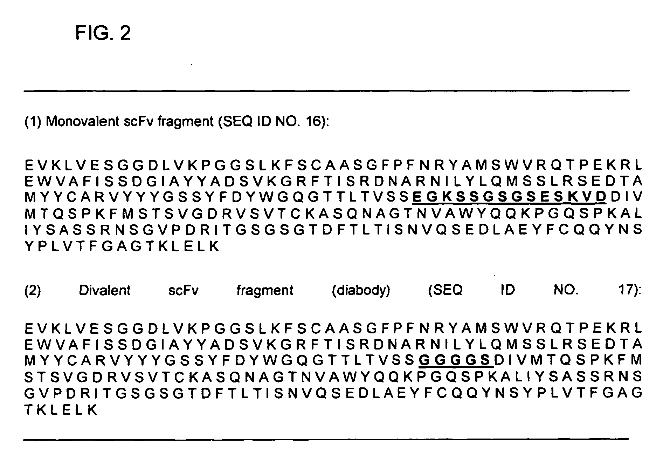

In FIGURE 2 the alignment of the aminoacid sequences (in one-letter code) deducted from the nucleotide ones for (1) the monovalent scFv fragment (SEQ ID No. 16), and (2) the divalent fragment (diabody) (SEQ ID No. 17), are presented. The order of the domains in both constructions are VH-linker segment-VL. The aminoacids of the linker segments employed in each of the two molecules appear in bold characters.

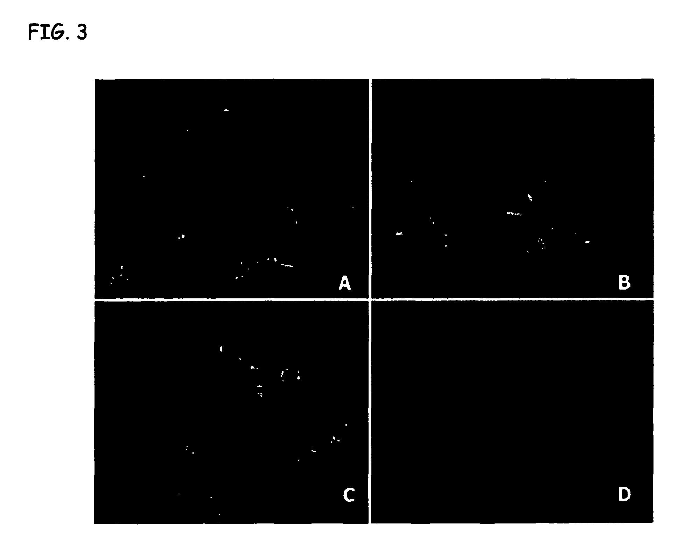

In FIGURE 3 the recognition of (A) Mab CB/ior-CEA.1, (B) monovalent scFv, and (C) diabody, for the CEA expressed in culture tumor cells AsPC-1 (ATCC CRL-1682) is exemplified, through the indirect immunofluorescence technique. In A, B, and C, the characteristic membrane and nearby cytoplasm fluorescence is seen. Magnification is 200x.

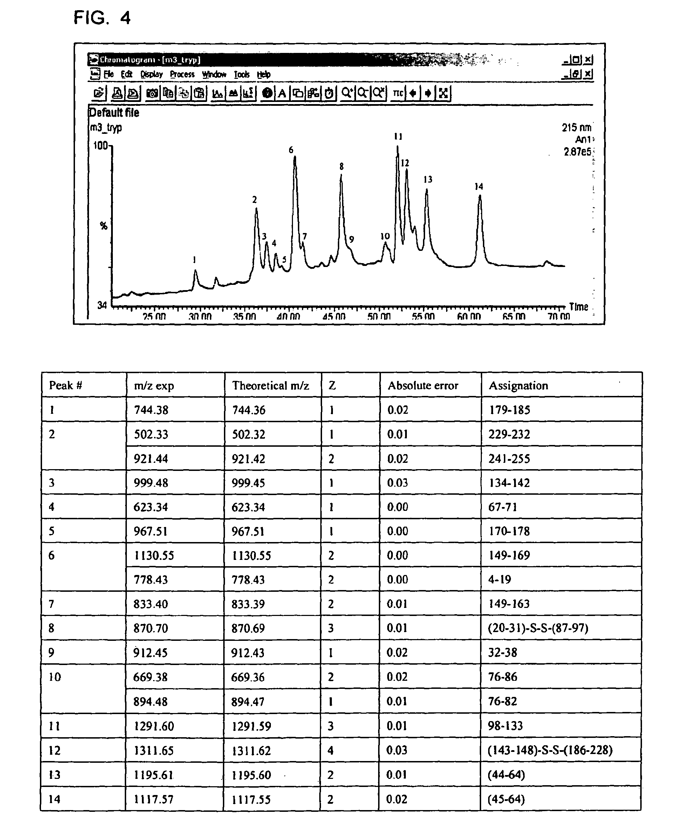

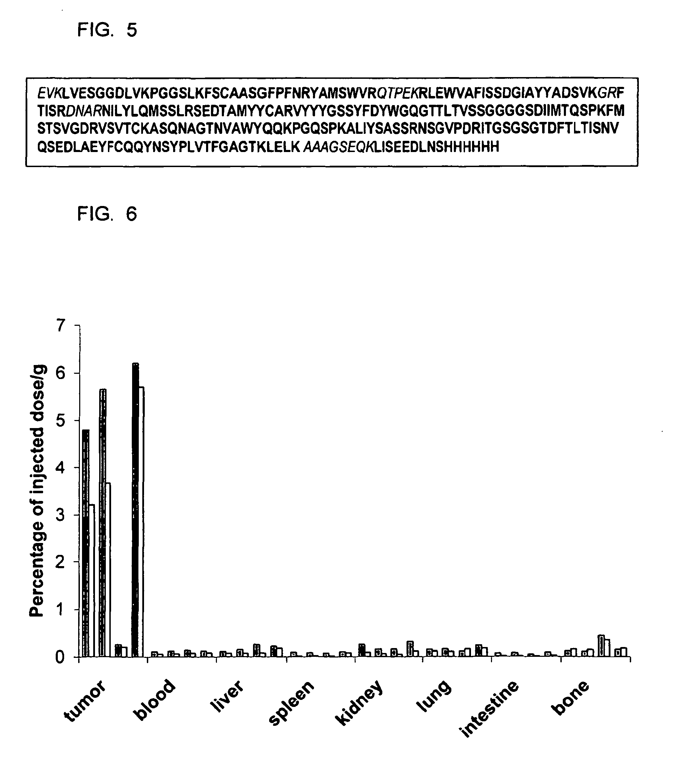

In FIGURE 4 the chromatographic profile of the proteolytic digestion of the diabody and the assignation of tryptic peptides obtained by mass spectrometry is presented. Up: Chromatographic profile of the tryptic digestion of the diabody. Down: Summary table of the assignation of the diabody tryptic peptides. M/z exp: experimental mass; theorical m/z: theoretical mass; Z: charge. In the obtained spectra signals corresponding to incorrectly linker cysteines were not detected. FIGURE 5 is a summary of the aminoacids sequence verification of the diabody (SEQ ID No. 21). The regions of the protein sequence that were verified by mass spectrometry are outlined in bold characters, and the zones of the sequence that were not recovered after tryptic digestion appear in italics. The zones in bold coincide in total with the aminoacid sequence deducted from the base sequence of the diabody. The sequence of the c-myc peptide and the final 6 histidines, provided by the pJG-1 m vector (FIGURE 1), are also seen in the C-terminus portion.

FIGURE 6 presents the percentage of the injected dose per gram of tissue, after 24 (stripe bars) and 48 (non-striped bars) hours of inoculation of mice bearing tumors that express human CEA with the following molecules radiolabeled with ¹²⁵I; from left to right, and in groups of four double bars: (a) diabody, (b) scFv, (c) F3, and (d) Mab CB/ior-CEA.1. Each bar represents the mean of the counts recovered from the organs obtained from 12 mice. The results demonstrate that between 24 and 48 hours, the ratio radioactivity in tumor: radioactivity in blood is maintained high for the diabody, the scFv, and Mab, with the highest values for the latter, followed by the dimeric molecule. F3 showed very low values, with an in vivo behavior inadequate that can be correlated by its diminished affinity for CEA.

### EXAMPLES

1. Amplification by PCR, cloning, and sequencing of the variable domains of Mab CB/ior-CEA.1
2. Assembly of scFv and diabody, expression in *E. coli,* and demonstration of their recognition of human CEA
3. Expression of the scFv and diabody in *Pichia pastoris* and demonstration of their recognition of human CEA
4. Purification of the scFv and diabody produced in bacteria
5. Characterization of the diabody through proteolytic digestion and mass spectrometry.
6. Studies of recognition of deglycosylated CEA
7. Immunocyto- and histo-chemical study in normal and tumor tissues.
8. Determination of the affinity constant.
9. Determination of the specific recognition in vivo of fragments and antibody labeled with ¹²⁵I, in C57BI/6 mice bearing tumors induced by the inoculation of B16-CEA13 cells.

### EXAMPLE 1. Amplification by PCR, cloning, and sequencing of the variable domains of the Mab CB/ior-CEA.1

### Procedure (a) Purification of RNA and amplification of variable regions

Total RNA from 10⁶ cells of the mouse hybridoma CB/ior-CEA.1 (Tormo B. et al. APMIS. 97: 1073-1080, 1989) was extracted with the TriPure^{™} reagent (Boehringer-Mannheim). The complementary DNA (cDNA) was synthesized using the First-Strand cDNA Synthesis for RT-PCR Kit (Boehringer-Mannheim), using oligo dT as primer. The polymerase chain reaction technique (PCR) for the specific amplification of the heavy and light chain variable domain genes was used. The employed synthetic primers were designed on the basis of the consensus sequences for mouse IgG and kappa chains, reported by Kabat E. et al. (US Department of Health and Human Services, NIH, 1991) and experiments developed previously in this laboratory (Coloma, MJ et al. Biotechniques 11: 152-156, 1991). The sequences of the oligonucleotides used in the PCR appear in Table I.

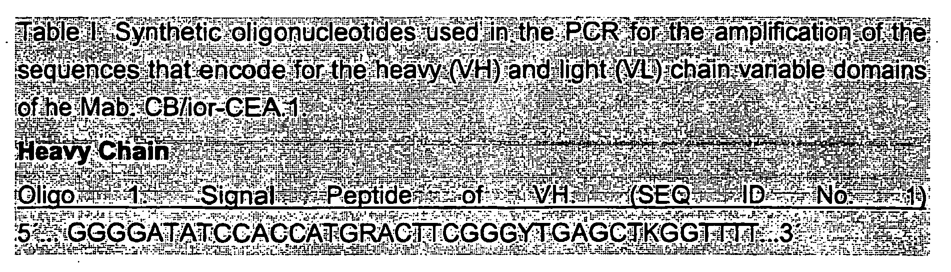

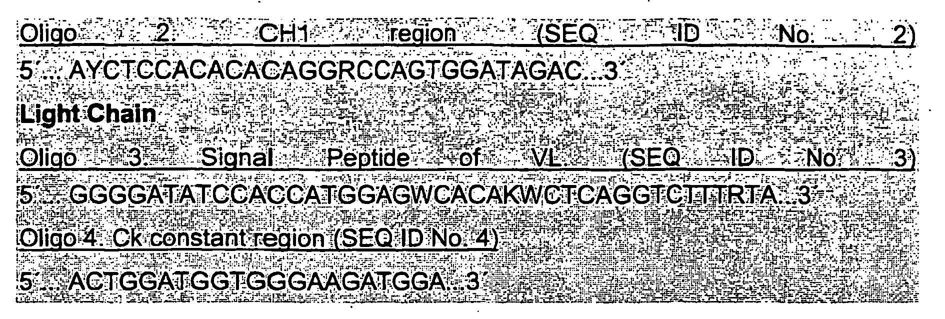

For PCR, the PCR Core Kit (Boehringer-Mannheim) was used. The conditions of the PCR were: denaturizing to 94°C, 1 minute, annealing to 55°C, 1 minute, extension to 72°C, 1 minute, 25 cycles, with 5 additional minutes of extension to the temperature already described in the last cycle, everything in a MJ Research Minicycler equipment. The final volumes of each reaction were 100 µL. All the oligonucleotides were used to a final concentration of 1 µM.

The DNA amplified fragments, witch the expected size of around 320-530 bp, were purified in low meeting point agarose gels (Sigma), using the QlAquick Gel Extraction Kit (QIAGEN, GmbH), and were cloned independently in the pMOS vector (Amersham Pharmacia Biotech), designed for the "blunt" cloning of DNA fragments.

### Procedure (b). Nucleotide sequence of the variable domains

For the determination of the nucleotide sequence of the light and heavy chain variables domains cloned in the vector pMOS, the oligonucleotides recommended by the manufacturer were used (Amersham Pharmacia Biotech). The base sequence was made by means of automatic methods, using an ALFexpress II equipment of Pharmacia (Amersham Biosciences), and the "Thermus Sequenase 5 Cy Dye Terminator Kit". The plasmids pVL2 and pVH5 were selected as representative of the sequences of VL and VH, respectively.

### EXAMPLE 2. Assembly of the scFv and diabody, expression in E. coli and demonstration of its recognition of human CEA

### Procedure (a). Re-amplification of the variable domains and assembly of scFv and diabody

The PCR was used for the assembly, in the form of scFv and diabody, of the VH and VL domains contained in the plasmids pVH5 and pVL2.

The synthetic oligonucleotides were designed on the basis of the sequences of VH and VL in the plasmids pVH5 and pVL2. These included sites of restriction for the cloning in the vector pJG-1 m and incorporated the linker segments of 14 and 5 aminoacids for the assembly of monomeric scFv and diabody (Tables II and III).

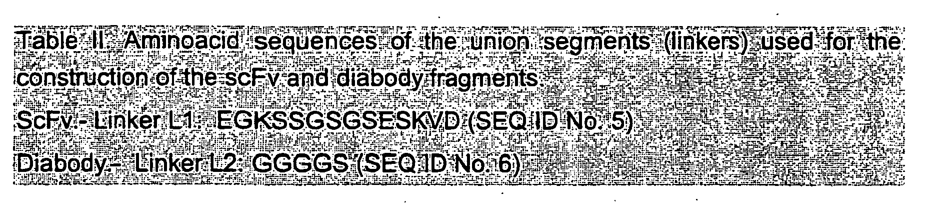

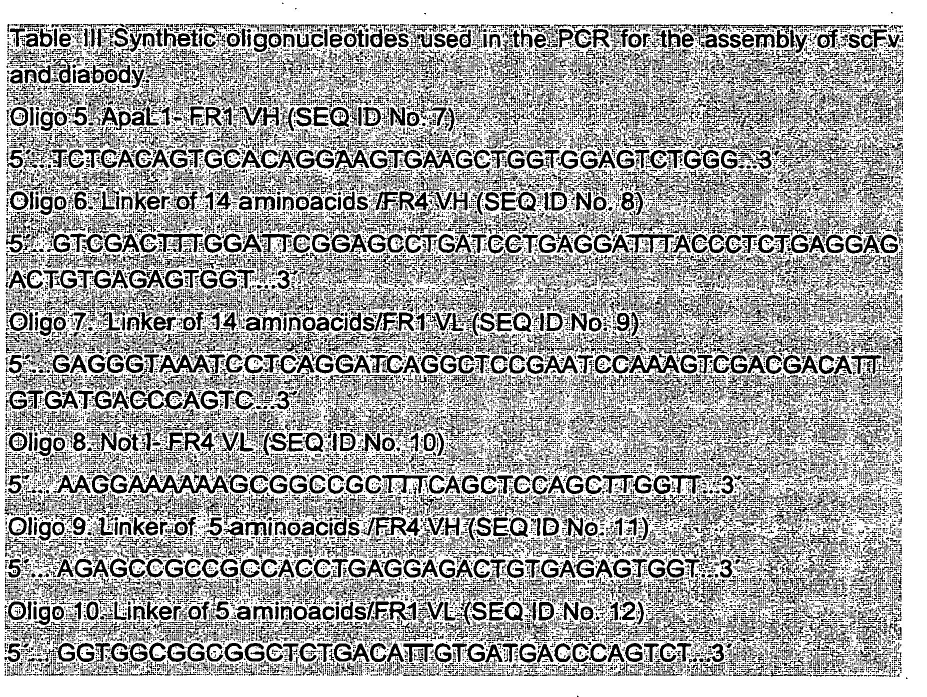

For the assembly of fragments, independent PCR were made in a first step to amplify:
1. For the domains that would give origin to the monovalent scFv. - Reaction 1: using the plasmid pVH5 as template with oligonucleotides 5 and 6 (Table III). Reaction 2: using the plasmid pVL2 with oligonucleotides 7 and 8 (Table III).
2. For the domains that would give origin to the diabody. - Reaction 3: using the plasmid pVH5 with oligonucleotides 5 and 9 (Table III). Reaction 4: using the plasmid pVL2 as template with oligonucleotides 8 and 10 (Table III).

The conditions and reagents used for the PCR were already described above. All the oligonucleotides were used to final concentration of 1 µM.

For the assembly of scFv a new PCR was made mixing 4 µL of reactions 1 and 2 with oligonucleotides 5 and 8 (Table III) in final concentration of 1 µM, and oligonucleotides 6 and 7 (Table III) in final concentration of 0.01 µM.

For the assembly of the diabody a new PCR was made mixing 4 µL of reactions 3 and 4 with oligonucleotides 5 and 8 (Table III) in final concentration of 1 µM, and oligonucleotides 6 and 7 (Table III) in final concentration of 0.01. µM.

The amplified DNA fragments were detected as majoritary bands of approximately 700 bp, and were isolated from low melting point agarose gels, as was described previously.

### Procedure (b). Cloning in pJG-1m vector.

The vector pJG-1 m is a plasmid designed for the expression of antibody fragments in the periplasm of *E. coli* (FIGURE 1). As main elements it has the LacZ promoter, a signal peptide, restriction sites ApaL I and Not I for the insertion of the fragment gene, a c-myc peptide and a sequence that encodes for 6 histidines. This last one is used as tag for the purification of expression products by immobilized metal ions affinity chromatography (IMAC; Porath J. Prot. Expr. Purif. 3: 263-281, 1992). The base sequences of the cloning restriction sites for the cloning of the scFv in question in the vector, and of the C-terminus amino acids that are added to the scFv, appear in FIGURE 1 (SEQ ID No. 13).

The DNA fragments corresponding to scFv and diabody, and the pJG-1 m vector were digested with ApaLI and Not I (Promega) restriction enzymes and the bands and vector ligated independently using T4 DNA ligase (Promega). The products of the ligation reactions were used for the transformation of competent *E. coli* (XL-1Blue strain; Stratagene) by electroporation, and the transformed cells were grown in solid selective medium (LB agar, with 100 µg/mL of ampicillin) during 16 hours at 37°C. The used methods are described in Molecular Cloning, A Laboratory Manual, Second Edition. Sambrook, Fritsch, Maniatis. 1989.

The recombinant plasmids were selected after the purification of the plasmid DNA from several colonies (QIAGEN MiniPrep kit), and the corresponding checking by digestion with the restriction enzymes already described for expected ligation products. In the restriction analyses, bands of approximately 3.5 kb were obtained corresponding to the linearized vector, and bands of approximately 700 bp for the genes encoding the scFv and diabody antibody fragments.

Five clones of each construction were sequenced using specifically designed primers that hybridize externally to the cloning regions of the vector pJG-1m (Table IV), by means of previously described procedures.

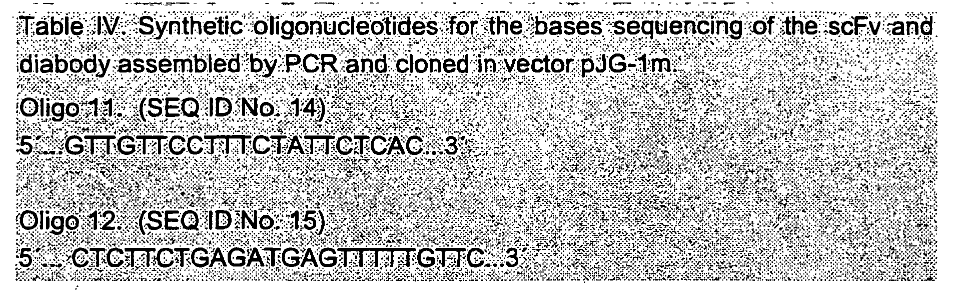

The aminoacid sequences derived from the base sequences obtained for the monovalent scFv (clone pJG1m-25) and the diabody (clone pJG1m-18) appear in FIGURE 2 (SEQ ID No. 16 and SEQ ID No. 17). With respect to a scFv developed previously (Ayala M et al. Biotechniques 13: 790-799, 1992), the VH and VL sequence now obtained for the new monovalent scFv and diabody exhibit 16 different aminoacids within the VH FR1, CDR2 and FR3 domains, and 3 different aminoacids within the VL FR1 and FR3 domains.

These results indicate that the \variable domains amplified and cloned from hybridoma CB/ior-CEA.1 to construct the new monovalent scFv and diabody can come from RNA different with respect to the ones used in the amplifications for the clonings of the previously reported scFv.

The linker segment of the new monovalent scFv is identical to the one of the scFv reported previously. The segment of union of the new scFv divalent (diabody) is different from the one of the scFv obtained previously, because it only includes 5 amino acids. In these experiments the sequences of the linker segments L1 and L2 were also verified, that appear in Table II.

### Procedure (c) Verification of the expression in E. coli of the scFv and diabody by SDS-PAGE and Western blot.

Competent *E. coli* cells TG1 were transformed independently with plasmids pJG1m-25 and pJG1m-18, containing the information for both antibody fragments. This strain allows the periplasmatic expression of the heterologous protein, or its secretion towards the culture medium.

The transformed bacteria were plated on solid selective medium and grown at 37°C for 16 hours. A representative colony of each of the two constructions were grown on liquid medium until OD₅₃₀ₙₘ = 1 and induced for 12 hours adding 1mM of IPTG to the culture medium. The cells were centrifuged and the periplasmatic content isolated by osmotic shock and brief sonication (seconds) for its evaluation in electrophoresis in 12% SDS-polyacrilamide gels (SDS-PAGE). This test revealed the expression in both cases of proteins of the expected molecular size (approximately 27 kDa), that were later evaluated by Western Blot using as primary antibody a Mab (9E10) specific against the peptide derived from c-myc that this protein contains (1 µg/mL), followed by rabbit anti-mouse IgG antibodies conjugated with horseradish peroxidase (Sigma). The transference of proteins from the SDS-PAGE to Hybond C Extra nitrocellulose (Amersham Life Sciences) was done in a semi-dry transference equipment (BioRad). DAB (Sigma) insoluble substrate was used in the development.

For the two constructions, the recombinant proteins with the mentioned size were identified with the Mab 9E10.

### Procedure (d) Specific recognition of the human CEA by scFv and diabody by ELISA

An ELISA test was made coating polyvinyl plates (Costar, 96-well Vinyl Assay Plates) with human CEA (Calbochem 219369), to a concentration of 1µg/mL. After blocking the plates with skim milk, the bacterial periplasm samples corresponding to the two constructions were added in dilutions of 1:5, 1:10, and 1:20 in PBS-2% skim milk, and incubated by 2 hours at room temperature.

For the detection of the union of fragments to the CEA, Mab 9E10 (1 µg/mL) was used, followed by mouse anti mouse IgG antibodies conjugated with horseradish peroxidase from Sigma. After several washings, OPD (Sigma) and H₂0₂ as chromogen and substrate were used to developl the reactions, and the quantitative evaluation of the reactions read at 492 nm in a LabSystems Multiskan MS.

In the test, Mab CB/ior-CEA.1 was used as positive control. Periplasm fractions corresponding to cells TG1 transformed with the vector pJG-1m without insert, and a non-related Mab, were used as negative controls. Also, plates were coated with the following irrelevant antigens: 10 µg/mL of bovine seroalbumine (BSA, Sigma), 10 µg/mL of ovalbumine, 10 µg/mL of lyzozyme, 10 µg/mL of keyhole limpet haemocyanin (Sigma). In all the plates wells were included where only phosphate buffered saline solution (PBS) without antigen (blank) was placed.

Values of absorbance at least 4 times greater than the produced by the negative controls were considered positive.

In these experiments the samples of periplasm of the constructions of scFv and diabody resulted positive with respect to their capacity of recognition of human CEA adsorbed to polyvinyl plates. These same samples were negative for all the irrelevant antigens.

### Procedure (e) Recognition of human CEA associated to cells by the scFv and diabody in ELISA and indirect immunofluorescence

The human tumor cell lines LoVo (ATCC CCL-229), AsPC-1 (ATCC CRL-1682), and LS 174T (ATCC CL-188), all which expresses CEA in culture, were seeded in 96 wells polystyrene plates (Costar). Once the confluence was reached, the wells were washed twice with PBS, drained off, and air-dried. The cells were then fixed to the plastic by using a 1:1 (v:v) mixture of cold acetone-methanol, for 3 minutes. After several washings with distilled water to eliminate residues, the plates were used as solid phase in ELISA tests where the samples of bacterial periplasm corresponding to the two constructions were added in dilutions of 1:2, 1:8, and 1:16, in PBS-2% skim milk, and incubated for 2 hours at room temperature. After several washings, Mab 9E10 (1 µg/mL) was used, followed by anti mouse IgG antibodies conjugated with horseradish peroxidase (Sigma) for the detection of the union of fragments to CEA. After several washings, the chromogen OPD (Sigma) and H₂0₂ as substrate were used to develop the reactions, and a LabSystems Multiskan. MS reader employed for quantitative evaluation of the reactions at 492nm. For the reading step, the supernatants were transferred to a fresh plate. The Mab CB/ior-CEA.1 was used as positive control in the assay. Periplasm fractions corresponding to cells TG1 transformed with the vector pJG-1 m without insert, and a non-related Mab, were used as negative controls. A plate with human cells HEK 293 (ATCC CRL-1573), that do not express CEA, was also used as negative control. The criteria of positivity were similar to the ones used in the ELISA described in the previous Procedure.

In this experiment the periplasm samples of the scFv and diabody constructions only recognized LoVo, AsPC-1 and LS 174T cells. All the negative controls were negative. In this way, the capacity of the scFv and diabody to identify the human CEA on human tumor cells that express this antigen, fixed on polystyrene plates, by cell-ELISA, was demonstrated.

In another experiment, LoVo, AsPC-1 and LS 174T cells were seeded in 35 mm diameter polystyrene plates (COSTAR) and cultivated until the confluence was reached. The plates were washed twice with PBS, drained off, they air-dried, and the cells fixed to the plastic using a 1:1 (v:v) mixture of cold acetone-methanol. After several washings with distilled water to eliminate residues, the plates were used as solid phase in indirect immunofluorescence tests. For this, circular zones were defined in the surface with fixed cells, in which bacterial periplasm samples corresponding to the two constructions, in dilutions of 1:2, 1:4 and 1:8 with PBS-3% BSA, were incubated independently. The same positive and negative controls used in the cell-ELISA were employed.

The incubation was at room temperature (RT) for 1 hour in humid chamber, followed by several washings with cold PBS-3% BSA, and the addition of Mab 9E10 (10 µg/mL) to all the monolayer by one hour at RT, also in humid chamber. After several washings with cold PBS- 3% BSA, the monolayer was incubated with anti mouse IgG antibodies conjugated with fluorescein isothiocyanate (FITC, Sigma) diluted 1:64 in PBS-3% BSA, for 30 minutes, in the dark and humid chamber, then were washed five times with PBS-3% BSA, once more with PBS, and finally stained with Evans Blue solution for a few minutes.

The monolayer was covered with PBS-10% glycerol, sealed with a cover slip and examined with a fluorescent light accessory Olympus BH2-RFL, mounted in an Olympus BHT microscope. Plates with HEK 293 human cells were also used as negative control. The presence of membrane and cytoplasm apple-green fluorescence was established as criterion of positive result, as long as this would not exist in the negative control samples, or in the human cells negative for CEA. In this experiment, the periplasm simples of the scFv and diabody constructions only recognized the LoVo, AsPC-1 and LS 174T cells. The negative controls were negative. In this way, the capacity of the scFv and diabody to identify the human CEA on human tumor cells that express this antigen, fixed on polystyrene plates, by indirect immunofluorescence, was demonstrated. An example of the results is shown in FIGURE 3.

### EXAMPLE 3. Expression of scFv and diabody in Pichia pastoris and demonstration of its recognition of human CEA

Procedure (a) Re-amplification of scFv and diabody and cloning in the vector pPS7.

The genes that codify for the scFv and diabody were amplified by PCR using as templates the constructions pJG1-25 and pJG1-18, respectively, and oligonucleotides designed to add the Ncol site in the 5' and 3' ends of the genes (Oligos 13 and 14; Table V), with the purpose of cloning in the *Pichia pastoris* expression vector pPS7. The amplification procedure was similar to the one described previously. Plasmid pPS7 is an integrative vector that contains a fragment of 1.15Kb that corresponds to the promoter of the alcohol oxidase (AOX.1) enzyme followed by the gene that codifies for the secretion signal of the sucrose invertase (sucll) of *Saccharamyces cerevisae,* a unique Ncol cloning site, a fragment of 960 bp of the enzyme glyceraldehyde 3-phosphate dehydrogenase (Gapt) to guarantee the completion of the transcription, and the HIS3 gene of *Saccharamyces cerevisae* as selection marker. In addition this vector contains a fragment of 2.1 kb, corresponding to the 3' sequence of the AOX.1 gene. All these elements are inserted in a vector pUC18 (Herrera Martinez LS et al., EP0438200 A1).

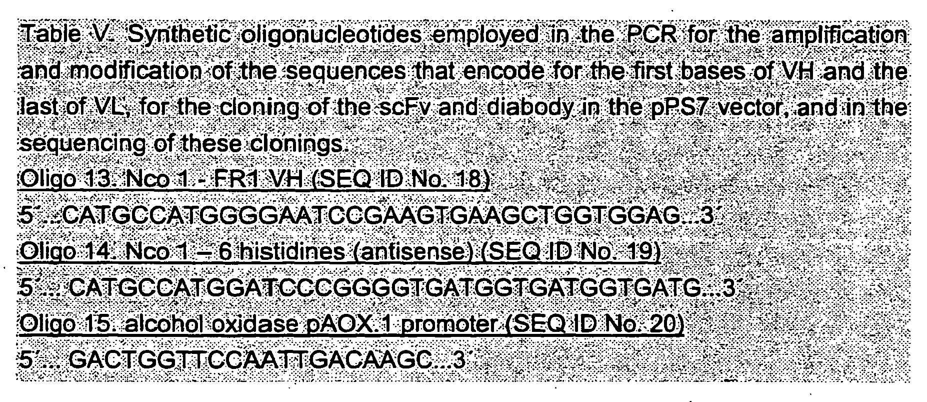

After the NcoI digestion (Promega) of the amplified bands corresponding to the scFv and diabody, these were ligated independently to the vector pPS7 previously digested with the same enzyme, and the products of the ligation were used to transform in an independent way the XL-1 Blue strain of *E*. *coli.* Isolated colonies corresponding to the transformation of the strain with each recombinant vector were analyzed using colony PCR with a primer that hybridizes in the promoter (Oligo 15, Table V) and another for the 3'end of VL (Oligo 8, Table III). Colonies that contain the correct insert oriented were selected. The sequencing of the cloned genes was made according to the previously described procedure (EXAMPLE 1 Procedure b), using Oligo 15 (Table V). The sequences obtained for the VH and VL domains of the recombinant plasmids pPSM2 (scFv) and pPSM3 (diabody) agreed with the previously cited in SEQ ID No. 16 and SEQ No. 17.

Recombinant strains of *Pichia pastoris* were obtained with these two plasmids through the electroporation of the MP36 his 3 wild strain (Yong V ET to. Biotechnol. Applic. 9: 55-61, 1992) with both mentioned plasmids, previously digested with the restriction enzyme Pvull (Promega), and selecting on histidine deficient minimum medium. As a result of the different recombination mechanisms of the recombinant plasmids with specific sites in the genome of *Pichia pastoris,* it was possible to isolate for each construction two different types of phenotypes of secretory strains: (a) strains in which the AOX.1 gene was not affected during the recombination event and therefore grew in media with methanol and showed to growth similar to the wild strain (Mut+), and (b) strains in which the AOX.1 gene was replaced by the expression cassette and showed slow growth in the presence of methanol (Mut s).

### Procedure (b) Expression studies

The studies of antibody fragment expression were made starting from the prototrophic colonies His+ grown in plates with selective MD medium (nitrogen yeast base, biotin, dextrose). The selected colonies were inoculated in 10 mL of rich BMGY buffered medium (yeast extract, peptone, potassium phosphate, nitrogen yeast base, biotin, and glycerol) in 50 mL tubes, and were placed at 28°C with rotation at 150 rpm. When the cultures reached 2 units of OD 600nm, measured in a SPECTRONIC GENESIS 2 equipment, these were centrifuged at 2000 rpm, during 10 minutes. The cellular pellets were suspended in 10 mL of rich medium with methanol (BMMY) as unique carbon source, instead of glycerol. From this moment on and during 96 hours the proteins of interest were induced, with daily addition of pure methanol until a final concentration of 1% in the culture. As negative control the MP36his3 strain transformed with a vector without insert was used.

Finalized the period of culture, the cells were centrifuged, the culture medium metabolized during the phase of induction collected, centrifuged once again for its final clarification and detection of scFv or diabody done by electrophoresis in 15% gels of SDS-polyacrilamide (SDS-PAGE). This test revealed the expression of proteins of the expected molecular weight in both cases (approx 27 kDa), that were later evaluated by Western Blot using the Mab 9E10 as primary antibody, and rabbit anti-mouse IgG antibodies conjugated with horseradish peroxidase (Sigma) as secondary antibody. The transferences were made as described above. In the development, DAB (Sigma) was used as insoluble substrate. For the two constructions, the recombinant proteins were identified with the Mab 9E10.

### Procedure (c) Recognition of the human CEA by scFv and diabody in ELISA.

An ELISA test, very similar to that previously described for the material derived from *E*. *coli,* was made using similar solid phases, reagents and coating, incubation, development, and positive control conditions. The samples of metabolized culture of the induced recombinant strains were diluted in PBS-1 % milk and added at the rate of 100 µL/well, and incubated for 2 hours at room temperature. As negative controls, metabolized medium corresponding to the strain MP36his 3, and a unrelated Mab, were used. Values were considered positives when absorbance was at least 4 times greater than that produced by the negative controls.

In this experiment the samples of induction-phase metabolized medium of the scFv and diabody constructions expressed in *Pichia pastoris* were positive with regards to their recognition capacity of human CEA adsorbed to polyvinyl plates.

### Procedure (d) Recognition of the human CEA associated to cells by cell-ELISA and indirect inmunofluorescence.

An ELISA test, very similar to that previously described for the material derived from *E*. *coli,* was made using similar solid phases, reagents and coating, incubation, development, and positive control conditions. The samples of metabolized culture of the induced recombinant strains were diluted in PBS-2% milk and added to the plates with fixed LoVo, AsPC-1, and LS 174T cells, and incubated for 2 hours at room temperature with gentle stirring. In the test, Mab CB/ior-CEA.1 was used as positive control. Metabolized induction phase cultures of strain MP36his transformed with the vector pPS7 without insert, and an unrelated Mab, were used as negative controls. Also, as negative control, a plate with human HEK 293 cells was used.

In this experiment the capacity of the scFv and diabody for specific identification of human CEA on human tumor cells fixed on polystyrene supports, by ELISA, was demonstrated.

An indirect immunofluorescence test, very similar to the previously described for the material derived from *E*. *coli,* was used, employing similar culture cells, and conditions of fixation, reagents, incubation, development, mounting, microscope observation and positive criterion. Independent zones were defined to the plates with fixed LoVo, AsPC-1 and LS 174T cells, in which the samples of induced cultures of the recombinant stocks corresponding to the two constructions, and negative ones, diluted in PBS - 3% BSA, 0.02 % sodium azide were applied.

The incubation was done at room temperature (RT) for 1 hour in humid chamber, followed of several washings with cold PBS-BSA-sodium azide, and the addition of Mab 9E10 to all the monolayer for 1 hour at RT, also in humid chamber. After several washings with cold PBS-3% BSA, the monolayer was incubated with anti mouse IgG antibodies conjugated with fluorescein isothiocyanate (Sigma) diluted 1:64 in PBS-3% BSA for 30 minutes in the dark and humid chamber. The plates were washed five times with PBS 3% BSA, once with PBS, and finally stained with_Evans Blue solution for a few minutes. The monolayers covered with PBS-10% glycerol were mounted with coverslips and examined in the ultraviolet light microscope.

In the assay, Mab CB/ior-CEA.1 was used as positive control. As negative controls the induced culture medium of MP36his3 transformed with pPS7 without insert, and a unrelated Mab, were used. Also a slide with HEK 293 cells was used as negative control. In this experiment the samples of the induced cultures that secreted the recombinant scFv and diabody recognized only LoVo, AsPC-1 and LS 174T cells. The negative controls were negative. The capacity of the scFv and diabody produced in *Pichia pastoris* to identify human CEA on human tumor cells that express this antigen fixed on polystyrene plates, by indirect immunofluorescence, was demonstrated.

### EXAMPLE 4.-Purification of the scFv and diabody produced in bacteria.

### Procedure (a) Purification of the scFv and diabody fragments, using immobilized ion metal affinity chromatography (IMAC) and ionic exchange

The presence of the six histidines domain in the recombinant protein, donated by the vector pJG-1m, was used for purification. These sequences confer proteins a very high affinity for metallic ions (for example Zn⁺², Cu⁺², Ni⁺²) that can be chelated to different chromatographic supports, allowing an easy and reproducible purification.

The recombinant bacteria obtained as described before were centrifuged and the periplasm contents isolated through osmotic shock and brief sonication (seconds), and after dialyzed for 72 hours in coupling buffer (Tris-HCl 20 mM, 1 M NaCl, 20mM Imidazole, pH 7.0). The bacterial periplasm preparations containing the scFv and the diabody were applied directly and independently to a Sepharose-IDA-Cu⁺² matrix (Pharmacia). Once the proteins were coupled, the gels were first washed with 10 times their volume using coupling buffer, followed in a similar fashion with wash buffer (Tris-HCl 20 mM, 1 M NaCl, 150 mM Imidazole, pH 7.0) to eliminate *E. coli* contaminant proteins. The elution of the scFv and the diabody was done with Tris-HCl 20mM, 1 M NaCl, 250 mM Imidazole, pH 7.0. The samples of the elution peaks were submitted to a 12% SDS-PAGE to verify the presence of the proteins of interest. The eluted fractions containing the scFv and the diabody were concentrated in UltraFree 15 (Amicon) devices, were dialyzed in a buffer solution containing Tris-HCl 20 mM, pH 8.7, and were submitted to a second step of purification using ionic exchange. For this, the samples were applied to a Mono Q column (Pharmacia), and eluted through a linear NaCl gradient (0 to 1 M). The samples of the collected peaks were checked in 12% SDS-PAGE. The presence of the scFv and diabody at the expected sizes (approximately 27 kDa) was verified. The final achieved purity for the two molecules was very similar and close to 95%, estimated through SDS-PAGE and silver staining. The peaks of pure scFv and diabody were concentrated in UltraFree 15 (Amicon) devices up to 2 mg/mL. The biological activity of the purified preparations was verified using ELISA, following a procedure similar to the one described previously in this invention. All the samples were conserved at 4°C.

### Procedure (b) Analysis of the scFv and diabody through gel filtration

The scFv and diabody purified as described by the previous procedure were studied using molecular sieve chromatography to determine the homogeneity of the samples and the presence of multimers. Superdex 200 (Pharmacia) was used for this, and a conventional process of gel filtration in a HPLC equipment. It was determined that the scFv concentrated in a major peak of approximately 27 kDa, correspondent to a monomeric form. The diabody appeared mainly with a size of approximately 45 kDa, corresponding to a dimeric form.

### EXAMPLE 5.- Characterization of the diabody through proteolytic digestion and mass spectrometry.

The purified diabody was dialyzed overnight at 4°C against a buffer solution containing 1% NH₄HCO₃ at pH=8.3, containing Urea at a concentration of 2 mol/L. The dialyzed protein was digested with sequence grade trypsin (Promega) in an enzyme:substrate ratio of 1:50 during 4 hours at 37°C. The proteolytic digestion was arrested by acidification with equal volume of an aqueous solution of 1% trifluoro acetic acid, and stored at -20°C until the moment of analysis by liquid chromatography coupled to the mass spectrometer (LC-MS).

The tryptic digestions were separated by reverse-phase chromatography in a liquid chromatograph AKTA Basic (Amersham Pharmacia Biotech) using a linear gradient from 0% to 80% of solution B in 100 minutes. The solutions used to generate the gradient were: A: H₂O / TFA 0.05 % and B: Acetonitrile / TFA 0.05 %.

The fractions obtained during the proteolytic digestion were analyzed by mass spectrometry using electrospray ionization (ESI-MS), by the way of connecting on line with the chromatographic system a LC-MS hybrid mass spectrometer with orthogonal geometry QTOF-2 (Micromass Ltd.). During the LC-MS measurement, the mass spectra were acquired from 350 to 1800 in 0.98 seconds and using 0.02 seconds between each scanning. The mass spectrometer was calibrated with a saline solution composed by a mixture of sodium and cesium iodide. The voltages used in the cone and the capillary were of 50 and 3000 volts, respectively. The spectra were processed using the programs package MassLinx v 3.5 (Micromass Ltd).

In FIGURE 4 and its adjunct Table the chromatographic profile of the tryptic digestion of the diabody, and the summary of the assignation of the tryptic peptides of the diabody, can be seen. In the ESI-MS spectra no signals indicating incorrectly linked cysteines were detected, evident from the summaries of the fractions 8 and 12 of the Table adjunct to FIGURE 4, that contains the peptides (²⁰Phe-Arg³¹)-S-S-(⁸⁷Ser-Arg⁹⁷) and (¹⁴³Val-Lys¹⁴⁸)-S-S-(¹⁸⁶Ile-Lys²²⁸) linked by disulphide bonds (-S-S-) between cysteines 22 and 95, and 147 and 212, respectively.

From the peptides analyzed by ESI-MS, 92% of the diabody sequence could be obtained in a single proteolytic digestion (FIGURE 5). In this sequence there is a full coincidence with the aminoacid sequences deducted from the base sequence of the VH and VL domains (SEQ ID No. 16 and 17), amplified by PCR starting from the total RNA of the hybridoma that produces the Mab CB/ior-CEA.1, of the 5-aminoacid linker segment (SEQ ID No. 10), and in the C-terminal portion, of the sequence of the c-myc peptide and 6 final histidines, provided by vector pJG-1m (FIGURE 2).

### EXAMPLE 6.- Studies of recognition of deglycosylated CEA.

Human CEA (Calbochem) was enzymatically deglycosylated with the endoglycosidase PNGasa F (New England Biolabs) specific for N-glycosylation. The CEA was dissolved in phosphate buffer 20 mM pH 7.8 and denatured with SDS and 2-mercaptoethanol at 100°C for 5 min. NP-40 and 1 µL of PNGasa F were then added for 2 hours at 37°C. The control and deglycosylated samples were analyzed in SDS-PAGE with Coomasie blue staining resulting in a significant reduction of molecular size (close to 50%) after digestion with the endoglycosidase. A Western blot was carried out using (a) Mab CB/ior-CEA.1, (b) the purified divalent scFv (diabody) or (c) an anti-human CEA antiserum obtained in mouse, as primary antibodies, followed by polyclonal antibodies against the Fab of Mab CB/ior-CEA.1 conjugated to horseradish peroxidase for (a) and (b), and anti-mouse IgG antibodies conjugated to horseradish peroxidase for (c). The transference and development were similar as described previously in this invention for Western blots. The Mab CB/ior-CEA.1 and the diabody only recognized the non deglycosylated antigen. The polyclonal antiserum recognized CEA before and after deglycosylation.

Samples of native human CEA were analyzed through a Dot Blot system for recognition of specific lectins. The employed lectins were the *Sambucus nigra* agglutinin (SNA) and the *Maackia amurensis* agglutinin (MAA), specific for terminal syalic acid, linked alpha 2,6 and alpha 2,3, respectively. The lectins employed in these experiments had been conjugated with Digoxigenin, which is identified by an anti-Digoxigenin antibody labeled with alkaline phosphatase. The samples positive for the interaction of lectin-oligosaccharide were developed by reaction with a substrates specific for phosphatase (a mixture of 4-nitro blue tetrazolium chloride and 5-bromo-4-chloro-3-indolyl-phosphate). Fetuin was used in this experiment as positive control from both lectins. The native CEA was recognized by SNA and not by MAA, a fact that indicated a high prevalence of terminal syalic acids linked alpha 2,6.

The human CEA was then digested with the enzyme NANAsa II, and exoglycosidase (syalidase) able to hydrolyze the terminal syalic acids alpha 2,6. The products of digestion were separated in SDS-PAGE, and the study of their recognition was done by Western blot, using as primary antibodies the Mab CB/ior-CEA.1 and an anti-human CEA antiserum obtained in mouse. The results indicated that the native CEA control was recognized by both samples, while only the mouse anti-CEA antiserum recognized the CEA digested with NANAsa II.

### EXAMPLE 7.- Immunocyto- and histochemical study in human normal and tumor tissues.

The tissues study was done in samples selected from normal and tumor tissue archives, coming from autopsy material. A minimum panel of tissues was used to verify the recognition already described for Mab CB/ior-CEA.1 (Tormo B et al. APMIS 97: 1073-1080, 1989). The specimens included: carcinomas of the lung, skin, breast, cervix, esophagus and kidney, adenocarcinomas of colon, prostate, pancreas, gall bladder, small intestine and stomach, tumors of neural, hematopoietic and sarcomatous origin, as well as normal colon mucosa, and normal tissues as liver, kidney, lung, testicle, spleen and pancreas, including also blood cells.

The study was done following procedures previously reported (Tormo B et al. APMIS 97: 1073-1080, 1989), with some variations. The tissue specimens were fixed in 10% buffered formalin, dehydrated, cleared and embedded in paraffin according to routine procedures. The histopathology was evaluated in sections colored with hematoxilin-eosin. Consecutive sections of the blocks evaluated by histopathology were used for the immunoperoxidase technique.

Paraffin-free, re-hydrated sections were treated with 3% H₂O₂ for 30 minutes to block endogenous peroxidase, washed in phosphate buffered saline (PBS), and incubated with the samples, these diluted in PBS-1% bovine serum albumin (dilution buffer), for one hour. Then, the slides were incubated for 30 minutes with a 1:100 dilution of biotinylated polyclonal IgG rabbit antibodies, obtained by immunization with the Fab of Mab CB/ior-CEA.1, and finally for a similar time with a 1:500 dilution of a peroxidase-streptavidin complex (Amersham).

The examined simples were:
(a) Mab CB/ior-CEA.1 (positive control) at a concentration of 20 µg/mL
(b) *E. coli* purified scFv, as described in EXAMPLE 4, procedure (a), at a concentration of 50 µg/mL
(c) *E. coli* purified diabody, as described in EXAMPLE 4, procedure (a), at a concentration of 50 µg/mL
(d) The previously obtained scFv, denominated "F3" in these Examples (Ayala et al. Biotechniques 13: 790-799, 1992; Pérez L et al. Applied Biochem. Biotechnol. 24: 79-82, 1996), purified and at concentrations of 50 and 100 µg/mL

All dilutions were done in dilution buffer, and the incubations at room temperature, in humid chamber. Between steps, 3 washings of 1 minute each with dilution buffer or PBS were done. The immunoperoxidase reaction was developed via a 5-10 minute incubation with a solution that contained 3 mg of diaminobencidine, 5 mL of LPBS and 5 mL of 30% H₂O₂. The slides were counter-stained with Meyer's hematoxilin. The characteristic brown color reaction was registered as: negative or positive, in three increasing intensity levels (1+, 2+, 3+). In each slide the labeling was done with the sample in question, and in an adjoining zone with the dilution buffer as negative control.

For the studies in blood cells, the erythrocytes were first removed, and remnant white cells were applied on glass slides coated with gelatin, and fixed with acetone:methanol 1:1 (v:v). The rest of the technique was developed basically as described above.

The obtained results are summarized in Table VI, with respect to the studied tissue. The normal tissues studied (liver, kidney, lung, testicles, blood, spleen, pancreas) were not identified by the fragments or by the Mab. In the case of the colonic mucosa, and in coincidence with what had been obtained before for Mab CB/ior-CEA.1, the F3 scFv, and the new scFv and diabody, reacted exclusively with the luminal secretion products and in the apical zones of some glands. The intensity of the reaction in the case of the F3 scFv was lower, something that was also later seen for several tumors. In the case of the blood cells, the Mab, the scFv and diabody did not show reaction with normal lymphocytes and neutrophils, indicating the absence of an important cross reactivity with the NCA antigen. On the contrary, the F3 scFv showed some minor recognition of these cells.

The Mab, the scFv, and the diabody reacted with most of the tumors of gastrointestinal origin, and the strong labeling was observed in the majority of the cases both in the apical surface of tumor cells, and in the cytoplasm. None of these samples labeled tumors of hematopoietic and sarcomatous origin, or others derived from epithelium, exception made of a canalicular breast carcinoma, and a lung large cell carcinoma. In the well differentiated colon adenocarcinomas the labeling was intense in the apical zone of the cytoplasm, and in the luminal secretion products, while in the moderately and poorly differentiated adenocarcinomas the labeling was observed in all cytoplasm. Exception made of very few samples, the staining intensities were very similar for these three molecules.

In the case of F3, a general lowering of the staining intensity was seen, even though in some occasions, concentrations two times higher than those used for the scFv and diabody were employed. The lower intensity of staining could have caused that some samples identified by the other antibodies were not recognized by F3.

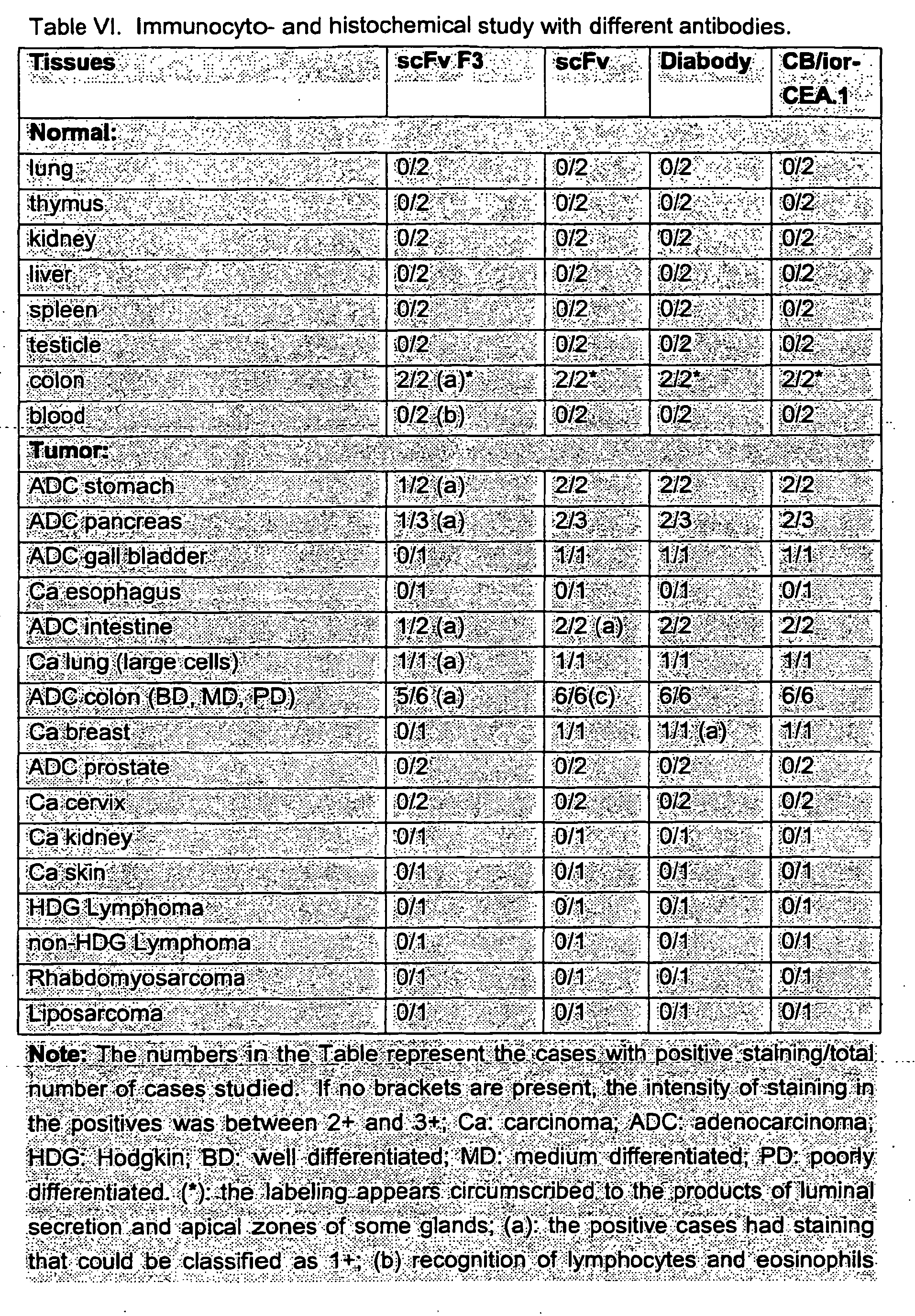

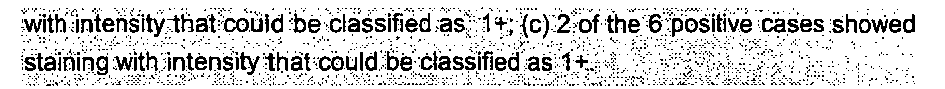

### EXAMPLE 8.- Determination of affinity constant.

For the determination of the affinity constant an ELISA no competitive method (Beatty JD et al. J. Immunol Meth. 100: 173-184, 1987) based on the mass action law, was used. The affinity constant Kaff is equal to [AgAb]/[Ag][Ab], where AgAb is the antigen-antibody complex in L/mol (M⁻¹), [Ag] is the concentration of free antigen (mol), and [Ab] is the concentration of free antibody (mol).

Four double serial dilutions of human CEA (Calbochem) were used in the coating of polyvinyl ELISA plates (Costar). The plates were blocked using PBS-skim milk 1%. The samples (scFv F3, scFv, diabody, Mab CB/ior-CEA.1, all purified) were applied to the plates at various concentrations. After washings, the wells corresponding to the three first samples were incubated with the Mab 9E10 (10 µg/mL), while in those corresponding to Mab CB/ior-CEA.1 blocking solution was used. In the following step an anti mouse IgG antibody conjugated to peroxidase (Sigma) was added in dilution 1:2500 for one hour, at 37°C. The used substrate was OPD, and the reaction was developed for 15 minutes. The reading of absorbance was done at 492 nm in a LabSystems Multiskan MS equipment.

The optical density (OD) values for each case were plotted in the ordinate axis (y), and the concentration in ng/mL in the abscissa axis (x), in a logarithm base 10 scale. OD 100 was taken as that at which the signal was maintained at a maximum. For each curve, half of the OD 100 (OD 50) was calculated. The concentration values of each sample at OD 50 were determined for each curve, and the affinity calculations carried out with the following formula: Kaff= (n-1)/2(n), where n= [Ab']t/[Ab]t. [Ab']t is the concentration value of the sample that corresponds to an OD 50 value for the highest antigen concentration to compare, and [Ab]t is the concentration value of the simple that corresponds to an OD 50 value for the lowest antigen concentration to compare. The six possible affinity determinations for the 4 obtained curves, estimating the final Kaff as the average of these.

Table VII reflects the Kaff values calculated for each the assayed variants. The scFv has a Kaff of (5.0 ± 0.4) x 10⁹ L mol⁻¹, a magnitude more that one order and a half higher than that obtained for F3 (Kaff= (9.2 ± 0.8) x 10⁷ L mol⁻¹). This last value basically corresponds with the calculated for F3 in measurements made by a different procedure (Pérez L et al. Applied Biochem. Biotechnol. 24: 79-82, 1996). The diabody has a Kaff of (2.8 ± 0.3) x 10¹⁰L mol⁻¹, while that for the Mab CB/ior-CEA.1 the Kaff value was (6.1 ± 0.5) x 10¹⁰ L mol⁻¹.

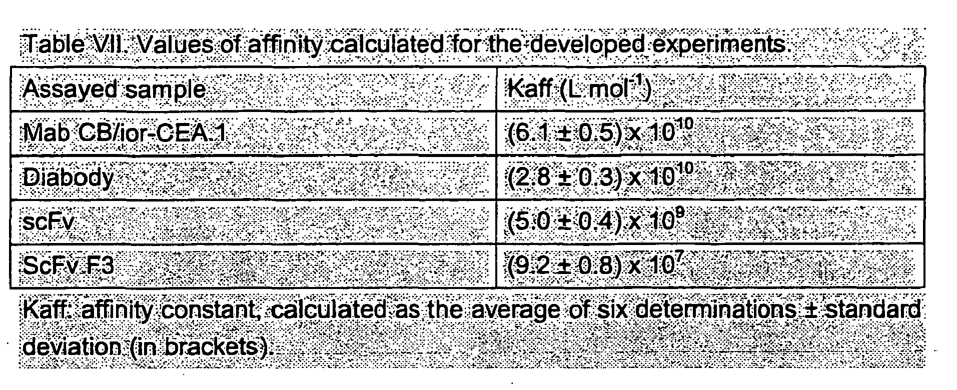

### EXAMPLE 9.- Determination of specific in vivo recognition of fragments and antibody labeled with ¹²⁵I, in C57BI/6 mice bearing tumors induced by the inoculation of B16-CEA13 cells.

For the determination of the *in vivo* specific recognition of the antibody fragments, the following molecules were labeled with ¹²⁵I (Amersham, UK) using the lodogen method (Fraker PJ, Speck JC Jr. Biochem Biophys Res Comm 80:849-857, 1978):
(a) scFv purified from *E*. *coli* ; (specific activity 1.1 MBq/5 µg)
(b) diabody purified from *E*. *coli* ; (specific activity: 1.2 MBq/5 µg)
(c) Mab CB/ior-CE4.1; (specific activity: 1.8 MBq/5 µg)
(d) Purified ScFv F3 (Ayala et al. Biotechniques 13: 790-799, 1992; Pérez L et al. Applied Biochem. Biotechnol. 24: 79-82, 1996) (specific activity: 1.0 MBq/5 µg).

The radiolabeled products were analyzed in thin layer chromatography to determine the incorporation to protein, and values between 95 and 98% of the radioactivity were found. The capacity of the radiolabeled products to detect CEA was assayed in a system where polystyrene tubes were coated with CEA (5 µg/mL; Calbochem), blocked, and samples of the radiolabeled products added, adjusted to the amounts of antibody that could be entrapped by this solid phase. After incubation a washing, it was determined that 80, 79, 83, and 81% of the radioactivity was entrapped by the solid phase, respectively, for the samples (a)-(d) described above, demonstrating that the radiolabeling procedure did not sensibly affect the biological activity of the antibodies.

To study the biodistribution, 4 groups of animals were formed, each of 12 C57BI/6 mice (CENPALAB, Cuba). The animals were inoculated with 1 x 10⁶ B16-CEA13 cells per animal, using the intra-axillary route. The tumors were visible and palpable (approximately 0.3-0.5 g) after 7 days, after which the mice were injected with the radiolabeled product in question by the tail vein, and sacrificed after 12, 24 and 48 hours, with surgical removal of the tumor and the following normal tissues: spleen, liver, kidney, intestine, muscle, bone marrow, and blood. The accumulation of radioactivity was expressed as percentage of the injected dose per gram of tissue. The calibration was done through a standard sample of the injected dose. Radioactivity was determined using a gamma scintillation counter.

The B16-CEA13 cells used in these experiments were obtained through the transfection of a gene that encodes for the extracellular domains of human CEA, cloned in the pDisplay™ vector (Cat. No. V660-20, Invitrogen). The gene was obtained by PCR from RNA extracted from CRL-1682 cells, with oligonucleotides designed alter the published sequence of human CEA. The recombinant plasmid pDisplay-CEA was purified and transfected into C57BI/6 mouse B16-F10 melanoma cells (ATCC CRL-6475) using Lipofectamine PLUS™ (Gibco-BRL) and 5 µg of DNA per transfection. The selection of stable transfectants was done with 4.0 mg/mL of geneticyn sulphate (G418; Gibco-BRL) for 14 days, after which the surviving cultured cells were cloned by limiting dilution and those clones that expressed human CEA in their surface were identified through indirect immunofluorescence, using Mab CB/ior-CEA.1 as first antibody, and anti mouse IgG antibodies conjugated with FITC (Sigma) for development. It was found that 73% of the clones presented more than 80% of the cells with specific membrane fluorescence indicating that the human CEA was exposed correctly folded and glycosylated in their surface.

B16-F10 non transfected cells were employed as controls. The replicas of the clones selected as positive through indirect immunofluorescence were multiplied and injected independently into C57BI/6 mice, 1 x 10⁶ cells per animal, using the intra-axillary route. Of the 10 clones that gave rise to tumors, the one with faster and progressive growth characteristics, denominated B16-CEA13, was selected for the experiments reported here.

FIGURE 6 shows the percentage of radioactivity recovered per studied tissue, at different times (with respect to the injected total), and the ratio radioactivity in the tumor:radioactivity in blood. The results included in Table VIII demonstrate that between 24 and 48 hours, the ratio radioactivity in the tumor:radioactivity in blood maintains high for the diabody, the scFv, and the Mab, with the highest values for the latter, followed by the dimeric molecule. The F3 scFv obtained previously showed very low values, with an in *vivo* inadequate behavior that can be correlated with its reduced affinity for CEA.

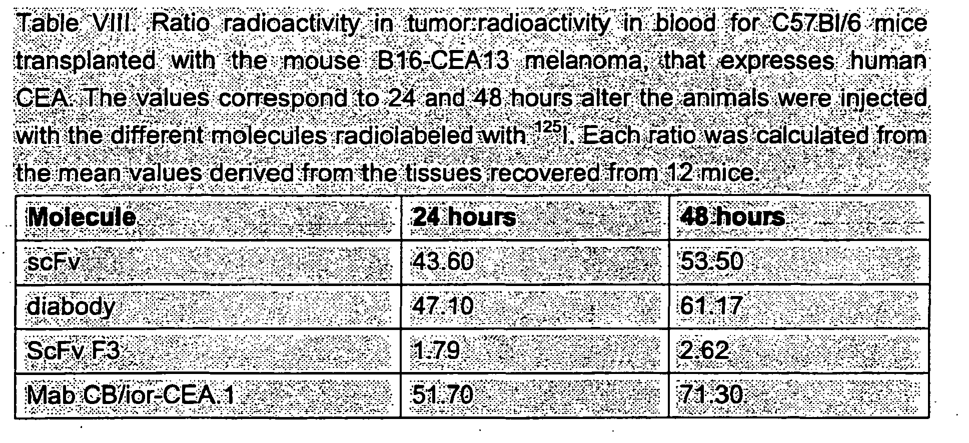

## Claims

1. A scFV antibody fragment specific for human carcinoembryonic antigen (CEA), having an antigen-binding site comprising the variable region of the light chain (VL) and the variable region of the heavy chain (VH) of the antibody as defined in SEX ID NO 16 or SEX ID NO 17 and wherein said variable region of the light chain (VL) and said variable region of the heavy chain (VH) are linked by a peptide linker.

2. Antibody fragment according to claim 1, wherein said scFv antibody fragment is a monovalent scFV and wherein said peptide linker has the amino acid sequence EGKSSGSGSESKVD.

3. Antibody fragment according to claim 1, wherein said scFV antibody fragment is a divalent scFV and wherein said peptide linker has the amino acid sequence GGGGS.

4. Antibody fragment according to any one of the preceding claims, wherein the recognition of human CEA is dependent on the conservation of CEA glycosylation.

5. Antibody fragment according to any one of the preceding claims, **characterized in that** they are produced in recombinant bacteria or yeast, in insect or mammalian transfected cells, or in non-human genetically modified organisms.

6. Antibody fragment according to any one of the preceding claims, **characterized in that** said fragment additionally contains a detectable label, or a chemical or biological agent with antitumor potential.

7. Pharmaceutical composition comprising an antibody fragment according to any one of claims 1-6, for the treatment of human tumors that express CEA.

8. Pharmaceutical composition comprising an antibody fragment according to any one of claims 1-6, for the *in vivo* radiolocalization of human tumors that express CEA, using imaging techniques.

9. Reagent for the *in vitro* or *ex vivo* diagnosis that contains an antibody fragment according to any one of claims 1-6, for the detection of human CEA, linked or not to cells.

10. Isolated cells that express an antibody fragment according to any one of claims 1-6, obtained through genetic manipulation by way of recombinant DNA, wherein said cells are bacteria, yeast, insect cells, mammalian cells, or plant cells.

11. Multicellular organism that expresses an antibody fragment according to any one of claims 1-6, obtained through genetic manipulation by way of recombinant DNA, wherein said organism is a transgenic non-human animal or transgenic plant.

12. Vectors that encode for an antibody fragment according to any one of claims 1-6, obtained through genetic manipulation by way of recombinant DNA, wherein these vectors are plasmids or sequences able to integrate in host cells.

13. An *ex vivo* method to determine the presence of a cell that expresses CEA, said method comprising providing a sample of cells and contacting said cells with an antibody fragment according to any one of claims 1-6 and determining the binding of the antibody fragment to the cells.

## Patentansprüche

1. Ein scFV Antikörper-Fragment, das spezifisch ist für das humane Carcinoembryonic Antigen (CEA), mit einer Antigen-Bindungs-Stelle, die die variable Region der leichten Kette (VL) und die variable Region der schweren Kette (VH) des Antikörpers umfasst, wie definiert wird in SEQ ID NO 16 bzw. SEQ ID NO 17, und wobei gesagte variable Region der leichten Kette (1/L) und besagte variable Region der schweren Kette (VH) verknüpft werden durch einen Peptid-Linker.

2. Das Antikörper-Fragment entsprechend Anspruch 1, wobei besagtes scFv-Antikörper-Fragment ein monovalentes scFV ist und wobei besagter Peptid-Linker die Aminosäure-Sequenz EGKSSGSGSESKVD aufweist.

3. Das Antikörper-Fragment entsprechend Anspruch 1, wobei besagtes scFV-Antikörper-Fragment ein bivalentes scFV ist und wobei besagte Peptid-Linker die Aminosäure-Sequenz GGGGS aufweist.

4. Das Antikörper-Fragment entsprechend irgendeinem der vorangegangenen Ansprüche, wobei die Erkennung von humanem CEA abhängig ist von der Konservierung der CEA-Glykosierung.

5. Das Antikörper-Fragment entsprechend irgendeinem der vorangegangenen Ansprüche, charakterisiert **dadurch**, dass es in rekombinanten Bakterien oder Hefe erzeugt wird, in Insekten- oder Säugetier-Transfektions-Zellen, oder in nicht-humanen genetisch modifizierten Organismen.

6. Das Antikörper-Fragment gemäß irgendeinem der vorangegangenen Ansprüche, charakterisiert **dadurch**, dass besagtes Fragment des weiteren einen nachweisbaren Label enthält oder ein chemisches oder biologisches Agens mit Antitumor-Potential.

7. Pharmazeutische Zusammensetzung umfassend ein Antikörper-Fragment gemäß irgendeinem der Ansprüche 1 bis 6 für die Behandlung von menschlichen Tumoren, welche CEA exprimieren.

8. Pharmazeutische Zusammensetzung, umfassend ein Antikörper-Fragment gemäß irgendeinem der Ansprüche 1 bis 6, für die *in vivo* Radiolokalisierung von menschlichen Tumoren, welche CEA exprimieren, unter Verwendung von bildgebenden Techniken.

9. Reagenz für die *in vitro* oder *ex vivo* Diagnose, welches ein Antikörper-Fragment enthält, gemäß irgendeinem der Ansprüche 1 bis 6, für den Nachweis von menschlichem CEA, verknüpft oder nicht an Zellen.

10. Isolierte Zellen, welche ein Antikörper-Fragment exprimieren gemäß irgendeinem der Ansprüche 1 bis 6, erhalten durch genetische Manipulation mit Hilfe rekombinanter DNA, wobei besagte Zellen bakterielle Zellen, Hefe- Zellen, Insekten-Zellen, Säugetier-Zellen oder Pflanzen-Zellen sind.

11. Multizellulärer Organismus, der ein Antikörper-Fragment gemäß irgendeinem der Ansprüche 1 bis 6 exprimiert, erhalten durch genetische Manipulation mit Hilfe von rekombinanter DNA, wobei besagter Organismus ein transgenes nicht-menschliches Tier oder eine transgene Pflanze ist.

12. Vektoren, die ein Antikörper-Fragment kodieren gemäß irgendeinem der Ansprüche 1 bis 6, erhalten durch genetische Manipulation mit Hilfe von rekombinanter DNA, wobei diese Vektoren Plasmide oder Sequenzen darstellen, welche in der Lage sind, in Wirtzellen zu integrieren.

13. Ein *ex vivo* Verfahren, um das Vorliegen einer Zelle zu bestimmen, welche CEA exprimiert, wobei besagtes Verfahren das Bereitstellen einer Probe aus Zellen und das In-Kontakt-Bringen von besagten Zellen mit einem Antikörper-Fragment gemäß irgendeinem der Ansprüche 1 bis 6 sowie das Bestimmen des Bindens des Antikörper-Fragments an die Zellen umfasst.

## Revendications

1. Fragment d'anticorps scFV spécifique de l'antigène carcino-embryonnaire (CEA) humain, comportant un site de liaison de l'antigène comprenant la région variable de la chaîne légère (VL) et la région variable de la chaîne lourde (VH) de l'anticorps tel que défini dans SEQ ID NO 16 ou SEQ ID NO 17 et dans lequel ladite région variable de la chaîne légère (VL) et ladite région de la chaîne lourde (VH) sont liées par un peptide lieur.

2. Fragment d'anticorps selon la revendication 1, ledit fragment d'anticorps scFv étant un scFV monovalent et dans lequel ledit peptide lieur a la séquence d'acides aminés EGKSSGSGSESKVD.

3. Fragment d'anticorps selon la revendication 1, ledit fragment d'anticorps scFV étant un scFV divalent et dans lequel ledit peptide lieur a la séquence d'acides aminés GGGGS.

4. Fragment d'anticorps selon l'une quelconque des revendications précédentes, la reconnaissance du CEA humain étant dépendante de la conservation de la glycosylation du CEA.

5. Fragment d'anticorps selon l'une quelconque des revendications précédentes, **caractérisé en ce qu'**il est produit dans une bactérie ou une levure recombinante, dans des cellules transfectées d'insectes ou de mammifères, ou dans des organismes génétiquement modifiés non humains.

6. Fragment d'anticorps selon l'une quelconque des revendications précédentes, **caractérisé en ce que** ledit fragment contient en plus un marqueur détectable, ou un agent chimique ou biologique avec un potentiel antitumoral.

7. Composition pharmaceutique comprenant un fragment d'anticorps selon l'une quelconque des revendications 1 à 6, pour le traitement de tumeurs humaines qui expriment le CEA.

8. Composition pharmaceutique comprenant un fragment d'anticorps selon l'une quelconque des revendications 1 à 6, pour la radiolocalisation *in vivo* de tumeurs humaines qui expriment le CEA, en utilisant des techniques d'imagerie.

9. Réactif pour le diagnostic *in vitro* ou *ex vivo* qui contient un fragment d'anticorps selon l'une quelconque des revendications 1 à 6, pour la détection de CEA humain, lié ou non à des cellules.

10. Cellules isolées qui expriment un fragment d'anticorps selon l'une quelconque des revendications 1 à 6, obtenues par manipulation génétique au moyen d'ADN recombinant, lesdites cellules étant des bactéries, des levures, des cellules d'insectes, des cellules de mammifères ou des cellules végétales.

11. Organisme multicellulaire qui exprime un fragment d'anticorps selon l'une quelconque des revendications 1 à 6, obtenu par manipulation génétique au moyen d'ADN recombinant, ledit organisme étant un animal transgénique non humain ou une plante transgénique.

12. Vecteurs qui codent un fragment d'anticorps selon l'une quelconque des revendications 1 à 6, obtenus par manipulation génétique au moyen d'ADN recombinant, ces vecteurs étant des plasmides ou des séquences capables de s'intégrer dans des cellules hôtes.

13. Procédé *ex vivo* pour déterminer la présence d'une cellule qui exprime le CEA, ledit procédé comprenant le fait de fournir un échantillon de cellules et de mettre en contact lesdites cellules avec un fragment d'anticorps selon l'une quelconque des revendications 1 à 6 et de déterminer la liaison du fragment d'anticorps aux cellules.


### SEQUENCE LISTING

<110> Center for Genetic Engineering and Biotechnology
<120> ANTIBODY FRAGMENTS SPECIFIC FOR HUMAN CARCINOEMBRYONIC ANTIGEN (CEA)
<130> Example of sequence listings
<140>01
   <141> 2002-04-03
<160> 21
<170> PatentIn Ver. 2.1
<210> 1
   <211> 39
   <212> DNA
   <213> Artificial Sequence
<220>
   <223> Description of Artificial Sequence: oligo
<400> 1
   ggggatatcc accatgract tcgggytgag ctkggtttt 39
<210> 2
   <211> 29
   <212> DNA
   <213> Artificial Sequence
<220>
   <223> Description of Artificial Sequence: oligo
<400> 2
   ayctccacac acaggrccag tggatagac 29
<210> 3
   <211> 40
   <212> DNA
   <213> Artificial Sequence
<220>
   <223> Description of Artificial Sequence: oligo
<400> 3
   ggggatatcc accatggagw cacakwctca ggtctttrta 40
<210> 4
   <211> 21
   <212> DNA
   <213> Artificial Sequence
<220>
   <223> Description of Artificial Sequence: oligo
<400> 4
   actggatggt gggaagatgg a 21
<210> 5
   <211> 14
   <212> PRT
   <213> Artificial Sequence
<220>
   <223> Description of Artificial Sequence: linker I
<400> 5
   Glu Gly Lys Ser Ser Gly Ser Gly Ser Glu Ser Lys Val Asp
   1 5 10
<210> 6
   <211> 5
   <212> PRT
   <213> Artificial Sequence
<220>
   <223> Description of Artificial Sequence: linker II
<400> 6
   Gly Gly Gly Gly Ser
   1 5
<210> 7
   <211> 39
   <212> DNA
   <213> Artificial Sequence
<220>
   <223> Description of Artificial Sequence: oligo
<400> 7
   tctcacagtg cacaggaagt gaagctggtg gagtctggg 39
<210> 8
   <211> 63
   <212> DNA
   <213> Artificial Sequence
<220>
   <223> Description of Artificial Sequence: oligo
<400> 8
   gtcgactttg gattcggagc ctgatcctga ggatttaccc tctgaggaga ctgtgagagt 60
   ggt 63
<210> 9
   <211> 62
   <212> DNA
   <213> Artificial Sequence
<220>
   <223> Description of Artificial Sequence: oligo
<400> 9
   gagggtaaat cctcaggatc aggctccgaa tccaaagtcg acgacattgt gatgacccag 60
   tc 62
<210> 10
   <211> 37
   <212> DNA
   <213> Artificial Sequence
<220>
   <223> Description of Artificial Sequence: oligo
<400> 10
   aaggaaaaaa gcggccgctt tcagctccag cttggtt 37
<210> 11
   <211> 36
   <212> DNA
   <213> Artificial Sequence
<220>
   <223> Description of Artificial Sequence: oligo
<400> 11
   agagccgccg ccacctgagg agactgtgag agtggt 36
<110> 12
   <211> 36
   <212> DNA
   <213> Artificial Sequence
<220>
   <223> Description of Artificial Sequence: oligo
<400> 12
   ggtggcggcg gctctgacat tgtgatgacc cagtct 36
<210> 13
   <211> 108
   <212> DNA
   <213> Artificial Sequence
<220>
   <223> Description of Artificial Sequence: vector
<400> 13
   cctttctatt ctcacagtgc acaggaaatc aaagcggccg cagggtccga acaaaaactc 60
   atctcagaag aggatctgaa ttcccatcat catcaccatc actaataa 108
<210> 14
   <211> 21
   <212> DNA
   <213> Artificial Sequence
<220>
   <223> Description of Artificial Sequence: oligo
<400> 14
   gttgttcctt tctattctca c 21
<210> 15
   <211> 24
   <212> DNA
   <213> Artificial Sequence
<220>
   <223> Description of Artificial Sequence: oligo
<400> 15
   ctcttctgag atgagttttt gttc 24
<210>
   <211> 241
   <212> PRT
   <213> Artificial Sequence
<220>
   <223> Description of Artificial Sequence: scFv
<400> 16
<210> 17
   <211> 232
   <212> PRT
   <213> Artificial Sequence
<220>
   <223> Description of Artificial Sequence: diabody
<400> 17
   Glu Val Lys Leu Val Glu Ser Gly Gly Asp Leu Val Lys Pro Gly Gly
   1 5 10 15
   Ser Leu Lys Phe Ser Cys Ala Ala Ser Gly Phe Pro Phe Asn Arg Tyr
   20 25 30
   Ala Met Ser Trp Val Arg Gln Thr Pro Glu Lys Arg Leu Glu Trp Val
   35 40 45
   Ala Phe Ile Ser Ser Asp Gly Ile Ala Tyr Tyr Ala Asp Ser Val Lys
   50 55 60
   Gly Arg Phe Thr Ile Ser Arg Asp Asn Ala Arg Asn Ile Leu Tyr Leu
   65 70 75 80
   Gln Met Ser Ser Leu Arg Ser Glu Asp Thr Ala Met Tyr Tyr Cys Ala
   85 90 95
   Arg Val Tyr Tyr Tyr Gly Ser Ser Tyr Phe Asp Tyr Trp Gly Gln Gly
   100 105 110
   Thr Thr Leu Thr Val Ser Ser Gly Gly Gly Gly Ser Asp Ile Val Met
   115 120 125
   Thr Gln Ser Pro Lys Phe Met Ser Thr Ser Val Gly Asp Arg Val Ser
   130 135 140
   Val Thr Cys Lys Ala Ser Gln Asn Ala Gly Thr Asn Val Ala Trp Tyr
   145 150 155 160
   Gln Gln Lys Pro Gly Gln Ser Pro Lys Ala Leu Ile Tyr Ser Ala Ser
   165 170 175
   Ser Arg Asn Ser Gly Val Pro Asp Arg Ile Thr Gly Ser Gly Ser Gly
   180 185 190
   Thr Asp Phe Thr Leu Thr Ile Ser Asn Val Gln Ser Glu Asp Leu Ala
   195 200 205
   Glu Tyr Phe Cys Gln Gln Tyr Asn Ser Tyr Pro Leu Val Thr Phe Gly
   210 215 220
   Ala Gly Thr Lys Leu Glu Leu Lys
   225 230
<210> 18
   <211> 35
   <212> DNA
   <213> Artificial Sequence
<220>
   <223> Description of Artificial Sequence: oligo
<400> 18
   catgccatgg ggaatccgaa gtgaagctgg tggag 35
<210> 19
   <211> 36
   <212> DNA
   <213> Artificial Sequence
<220>
   <223> Description of Artificial Sequence: oligo
<400> 19
   catgccatgg atcccggggt gatggtgatg gtgatg 36
<210> 20
   <211> 21
   <212> DNA
   <213> Artificial Sequence
<220>
   <223> Description of Artificial Sequence: diabody MS
<400> 20
   gactggttcc aattgacaag c 21
<210> 21
   <211> 255
   <212> PRT
   <213> Artificial Sequence
<220>
   <223> Description of Artificial Sequence: diabody MS
<400> 21
   Glu Val Lys Leu Val Glu Ser Gly Gly Asp Leu Val Lys Pro Gly Gly
   1 5 10 15
   Ser Leu Lys Phe Ser Cys Ala Ala Ser Gly Phe Pro Phe Asn Arg Tyr
   20 25 30
   Ala Met Ser Trp Val Arg Gln Thr Pro Glu Lys Arg Leu Glu Trp Val
   35 40 45
   Ala Phe Ile Ser Ser Asp Gly Ile Ala Tyr Tyr Ala Asp Ser Val Lys
   50 55 60
   Gly Arg Phe Thr Ile Ser Arg Asp Asn Ala Arg Asn Ile Leu Tyr Leu
   65 70 75 80
   Gln Met Ser Ser Leu Arg Ser Glu Asp Thr Ala Met Tyr Tyr Cys Ala
   85 90 95
   Arg Val Tyr Tyr Tyr Gly Ser Ser Tyr Phe Asp Tyr Trp Gly Gln Gly
   100 105 110
   Thr Thr Leu Thr Val Ser Ser Gly Gly Gly Gly Ser Asp Ile Ile Met
   115 120 125
   Thr Gln Ser Pro Lys Phe Met Ser Thr Ser Val Gly Asp Arg Val Ser
   130 135 140
   Val Thr Cys Lys Ala Ser Gln Asn Ala Gly Thr Asn Val Ala Trp Tyr
   145 150 155 160
   Gln Gln Lys Pro Gly Gln Ser Pro Lys Ala Leu Ile Tyr Ser Ala Ser
   165 170 175
   Ser Arg Asn Ser Gly Val Pro Asp Arg Ile Thr Gly Ser Gly Ser Gly
   180 185 190
   Thr Asp Phe Thr Leu Thr Ile Ser Asn Val Gln Ser Glu Asp Leu Ala
   195 200 205
   Glu Tyr Phe Cys Gln Gln Tyr Asn Ser Tyr Pro Leu Val Thr Phe Gly
   210 215 220
   Ala Gly Thr Lys Leu Glu Leu Lys Ala Ala Ala Gly Ser Glu Gln Lys
   225 230 235 240
   Leu Ile Ser Glu Glu Asp Leu Asn Ser His His His His His His
   245 250 255